# EUROPEAN PATENT SPECIFICATION

(11) **EP 2 576 826 B1**
(45) Date of publication and mention of the grant of the patent: **17.09.2014**
(21) Application number: 11725949.9
(22) Date of filing: 06.06.2011
(51) Int. Cl.: C12Q 1/68

(54) **METHODS AND MEANS FOR PREDICTING OR DIAGNOSING TYPE II DIABETES OR MYOCARDIAL INFARCTION BASED ON MICRO RNA DETECTION.**
VERFAHREN UND MITTEL FÜR DIE VORAUSSAGE DER DIAGNOSE VON TYP-2-DIABETES ODER MYOKARDINFARKT AUF BASIS EINES MICRO-RNA-NACHWEISES
MÉTHODES ET MOYENS DE PRÉVISION OU DE DIAGNOSTIC DU DIABÈTE DE TYPE II OU DE L'INFARCTION DU MYOCARDE REPOSANT SUR LA DÉTECTION DE MICRO ARN

(30) Priority: 07.06.2010 GB 201009513; 26.01.2011 GB 201101400
(43) Date of publication of application: 10.04.2013
(73) Proprietor: King's College London, London WC2R 2LS (GB)
(72) Inventor: MAYR, Manuel, London SE5 9NU (GB)
(74) Representative: Tombling, Adrian George
(86) International application number: PCT/GB2011/000850
(87) International publication number: WO 2011/154689

(56) References cited:
- WO-A1-2010/019574
- WO-A2-2009/143379
- US-A1- 2008 305 474
- US-A1- 2009 005 336
- CHEN L ET AL: "The role of microRNA expression pattern in human intrahepatic cholangiocarcinoma", JOURNAL OF HEPATOLOGY, MUNKSGAARD INTERNATIONAL PUBLISHERS, COPENHAGEN, DK, vol. 50, no. 2, 1 February 2009 (2009-02-01), pages 358-369, XP025949528, ISSN: 0168-8278, DOI: DOI:10.1016/J.JHEP.2008.09.015 [retrieved on 2008-11-21]
- P. MESTDAGH ET AL: "High-throughput stem-loop RT-qPCR miRNA expression profiling using minute amounts of input RNA", NUCLEIC ACIDS RESEARCH, vol. 36, no. 21, 21 October 2008 (2008-10-21), pages E143-E143, XP55003045, ISSN: 0305-1048, DOI: 10.1093/nar/gkn725
- KELLER ANDREAS ET AL: "miRNAs in lung cancer - Studying complex fingerprints in patient's blood cells by microarray experiments", BMC CANCER, BIOMED CENTRAL, LONDON, GB, vol. 9, no. 1, 6 October 2009 (2009-10-06) , page 353, XP021062692, ISSN: 1471-2407, DOI: DOI:10.1186/1471-2407-9-353
- WANG GUO-KUN ET AL: "Circulating microRNA: a novel potential biomarker for early diagnosis of acute myocardial infarction in humans", EUROPEAN HEART JOURNAL (ONLINE), OXFORD UNIVERSITY PRESS, GB, US, NL, vol. 31, no. 6, 1 March 2010 (2010-03-01), pages 659-666, XP002586116, ISSN: 1522-9645, DOI: DOI:10.1093/EURHEART/EHQ013
- HOEKSTRA MENNO ET AL: "The peripheral blood mononuclear cell microRNA signature of coronary artery disease", BIOCHEMICAL AND BIOPHYSICAL RESEARCH COMMUNICATIONS, ACADEMIC PRESS INC. ORLANDO, FL, US, vol. 394, no. 3, 1 April 2010 (2010-04-01) , pages 792-797, XP002586117, ISSN: 0006-291X, DOI: DOI:10.1016/J.BBRC.2010.03.075
- FASANARO P ET AL: "microRNA: Emerging therapeutic targets in acute ischemic diseases", PHARMACOLOGY AND THERAPEUTICS, ELSEVIER, GB, vol. 125, no. 1, 1 January 2010 (2010-01-01), pages 92-104, XP026812449, ISSN: 0163-7258 [retrieved on 2009-11-06]
- JASON E. FISH ET AL: "miR-126 Regulates Angiogenic Signaling and Vascular Integrity", DEVELOPMENTAL CELL, vol. 15, no. 2, 12 August 2008 (2008-08-12), pages 272-284, XP55002871, ISSN: 1534-5807, DOI: 10.1016/j.devcel.2008.07.008 cited in the application
- XI CHEN ET AL: "Characterization of microRNAs in serum: a novel class of biomarkers for diagnosis of cancer and other diseases", CELL RESEARCH CHINA,, vol. 18, no. 10, 1 October 2008 (2008-10-01), pages 997-1006, XP002634302, ISSN: 1748-7838, DOI: DOI:10.1038/CR.2008.282 [retrieved on 2008-09-02]
- SUN L L ET AL: "MicroRNA-15a positively regulates insulin synthesis by inhibiting uncoupling protein-2 expression", DIABETES RESEARCH AND CLINICAL PRACTICE, AMSTERDAM, NL, vol. 91, no. 1, 1 January 2011 (2011-01-01), pages 94-100, XP027574330, ISSN: 0168-8227 [retrieved on 2010-12-24]
- H. LU ET AL: "MicroRNA-223 regulates Glut4 expression and cardiomyocyte glucose metabolism", CARDIOVASCULAR RESEARCH, vol. 86, no. 3, 1 June 2010 (2010-06-01), pages 410-420, XP55003032, ISSN: 0008-6363, DOI: 10.1093/cvr/cvq010
- A. HE ET AL: "Overexpression of Micro Ribonucleic Acid 29, Highly Up-Regulated in Diabetic Rats, Leads to Insulin Resistance in 3T3-L1 Adipocytes", MOLECULAR ENDOCRINOLOGY, vol. 21, no. 11, 1 November 2007 (2007-11-01), pages 2785-2794, XP55003191, ISSN: 0888-8809, DOI: 10.1210/me.2007-0167
- ZAMPETAKI ANNA ET AL: "Plasma microRNA profiling reveals loss of endothelial miR-126 and other microRNAs in type 2 diabetes.", CIRCULATION RESEARCH 17 SEP 2010 LNKD- PUBMED:20651284, vol. 107, no. 6, 17 September 2010 (2010-09-17), pages 810-817, XP008139609, ISSN: 1524-4571

## Description

The present invention relates to a method of detecting diabetes and associated complications. The present invention also relates to a method of predicting diabetes. The present invention also relates to a method of predicting myocardial infarction.

MicroRNAs (miRNAs) are a class of small non-coding RNAs that function as translational repressors. They bind through canonical base pairing to a complementary site in the 3' untranslated region (UTR) of their target mRNAs and can direct the degradation or translational repression of these transcripts ¹⁻². MiRNAs have been shown to play important roles in development, stress responses, angiogenesis and oncogenesis ³⁻⁴. Accumulating evidence also points to an important role of miRNAs in the cardiovascular system ⁵⁻⁷.

Recently, Mitchell et al. highlighted the presence of miRNAs in plasma ⁸. These plasma miRNAs are not cell-associated, but packaged in microvesicles that protect them from endogenous RNase activity. Interestingly, plasma miRNAs can display unique expression profiles: specific tumour miRNAs were identified in cancer patients ⁹, while tissue-derived miRNAs constitute a marker for injury ¹⁰⁻¹¹. In cardiovascular diseases, circulating miRNAs have been investigated in sepsis, myocardial injury and heart failure¹²⁻¹⁴.

WANG et al. (Circulating microRNA: a novel potential biomarker for early diagnosis of acute myocardial infarction in humans, EUROPEAN HEART JOURNAL, vol. 31, no. 6, March 2010, pages 659-666) relates to miR expression pattern in plasma. In rats and human patients with acute myocardial infarction, plasma levels of miR-1, miR-133a, miR-499 and miR-208a increased. Elevated cardiac-specific miR-208a in plasma may be a biomarker for early detection of myocardial injury.

US 2009/005336 A1 relates to miR-1 for the treatment, prevention and diagnosis of cardiac conditions including myocardial infarction. Paragraph 5.1 p. 99, relating to diagnostic and prognostic mRNAs, mentions only the potential value of miRs in the cardiovascular field and the identification of miR-208 in rat model of myocardial injury.

WO 2010/019574 A1 relates to miR-126, regulator of vascular integrity, expressed in endothelial cells. Administering miR-126 agonist to promote vascular integrity or endothelial repair. miR-126 null mice show abnormalities during embryogenesis such as hemorrhages, ruptured blood vessels, and have reduced survival after myocardial infarction.

Type II diabetes mellitus (DM) is one of the major risk factors of cardiovascular disease leading to endothelial dysfunction and micro- and macrovascular complications ¹⁵⁻¹⁶. However, a systematic analysis of plasma miRNAs in DM has not yet been performed.

SUN et al. (MicroRNA-15a positively regulates insulin synthesis by inhibiting uncoupling protein-2 expression, DIABETES RESEARCH AND CLINICAL PRACTICE, vol. 91, no. 1, January 2011, pages 94-100) relates to miR-15a as a positive regulator of insulin synthesis.

LU et al. (MicroRNA-223 regulates Glut4 expression and cardiomyocyte glucose metabolism, CARDIOVASCULAR RESEARCH, vol. 86, no. 3, June 2010, pages 410-420) relates to miR-223 in the regulation of glucose transporter 4 (Glu4) and cardiomyocyte glucose metabolism.

HE et al. (Overexpression of Micro Ribonucleic Acid 29, Highly Up-Regulated in Diabetic Rats, Leads to Insulin Resistance in 3T3-L1 Adipocytes, MOLECULAR ENDOCRINOLOGY, vol. 21, no. 11, November 2007, pages 2785-2794) relates to miR-29 up-regulation in diabetic rats and its overexpression in adipocytes leading to insulin resistance.

There is a need for a miRNA signature for diabetes to allow the identification of individuals with diabetes, identify individuals at risk of developing diabetes and its associated complications, and also to monitor how effectively individuals are managing their diabetes.

Accordingly to a first aspect of the present invention there is provided a method of determining whether an individual has type II diabetes comprising determining in a sample obtained from the individual the level of the following microRNAs: miR-15a, miR-126, miR223, miR-320 and miR-28-3p.

It has been found that by making the determination set out above it is possible to determine with high specificity and sensitivity whether an individual has type II diabetes. Specificity is defined as the proportion of true negatives (individuals that are not diabetic) identified as non-diabetic in the method. Sensitivity is defined as the proportion of true positives (individuals that are diabetic) identified as diabetic in the method. The method provides a highly accurate test that can be performed relatively easily as only a few biomarkers (i.e., the microRNAs) are measured. A simple and effective test of whether an individual is diabetic is therefore provided.

The term "diabetes" as used herein is well known to those skilled in the art and refers to both type I and type II diabetes. Preferably, the term refers to type II diabetes. It is further preferred that the term refers to untreated or poorly treated type II diabetes. It has been found that diabetes that is well treated may not be detected using the present method. Accordingly, the method according to the first aspect of the invention can also be used to determine if a patient with type II diabetes is treating the condition effectively as the test will not show the variation in the levels of the mircroRNAs associated with diabetes when the individual is effectively being treated. The present method therefore enables one to monitor the effectiveness of diabetic treatments. An individual that is effectively being treated for diabetes is an individual that controls their fasting blood glucose levels to between about 3 and 8 mmols/litre, preferably between about 4 and 7 mmols/litre.

It has been found in individuals that have diabetes that the level of miR-15a, miR-126, miR223 and miR-320 is reduced, and the level of miR-28-3p is increased.

The method according to the first aspect of the present invention may additionally comprising determining the level of one or more of the following additional mircoRNAs: miR-20b, miR-21, miR-24, miR-29b, miR-191, miR-197, miR-486 and miR-150. Any combination of the additional microRNAs can be used. Although all 8 additional microRNAs may be used in making such a determination, preferably only 1, 2, 3, 4 or 5 additional microRNAs are used to make such a determination. It has been found in individuals that have diabetes that the level of all the additional microRNAs are reduced.

According to a second aspect of the present invention there is provided a method of predicting type II diabetes comprising determining in a sample obtained from an individual the level of the following microRNAs: miR-15a, miR-29b, miR-126, miR223 and miR-28-3p.

It has been found that by making the determination set out above it is possible to predict with high specificity and sensitivity whether an individual is likely to develop type II diabetes. Specificity is defined as the proportion of true negatives (individuals that do not develop diabetes) identified as not developing diabetes in the method. Sensitivity is defined as the proportion of true positives (individuals that develop diabetes) identified as developing diabetes in the method. The method provides a highly accurate test that can be performed relatively easily as only a few biomarkers (i.e., the microRNAs) are measured. A simple and effective test of whether an individual is likely to develop type II diabetes is therefore provided.

It has been found that a positive prediction of type II diabetes is given by a reduced level of miR-15a, miR-29b, miR-126 and miR223, and an increased level of miR-28-3p.

The method according to the first and second aspect of the present invention may additionally comprise determining the level of miR-454 and/or RNU6b (a small nuclear RNA) as a control. The levels of the controls should not differ significantly between individuals who have or are likely to develop diabetes and individuals who do not have diabetes and are not likely to develop diabetes.

The method according to the first aspect and second aspect of the present invention is performed on a sample obtained from the individual. The sample may be any suitable sample from which it is possible to measure the microRNAs mentioned above. Preferably the sample is blood, serum, plasma or other blood fractions, or a tissue sample. Most preferably the sample is a blood plasma sample.

miR-15a is a standard term well known to those skilled in the art. In particular, the sequence of the human form of miR-15a is given in the NCBI protein database under accession number NR_029485.1, version GI: 262205622.

miR-20b is a standard term well known to those skilled in the art. In particular, the sequence of the human form of miR-20b is given in the NCBI protein database under accession number NR_029950.1, version GI: 262205365.

miR-21 is a standard term well known to those skilled in the art. In particular, the sequence of the human form of miR-21 is given in the NCBI protein database under accession number NR_029493.1, version GI: 262205659.

miR-24 is a standard term well known to those skilled in the art. In particular, the sequence of the human form of miR-24 is given in the NCBI protein database under accession number, NR_029496.1, version GI:262205676.

miR-28-3p is a standard term well known to those skilled in the art. In particular, the sequence of the human form of miR-28-3p is given in the NCBI protein database under accession number 029502.1, version GI: 262205701.

miR-29b is a standard term well known to those skilled in the art. In particular, the sequence of the human form of miR-29b is given in the NCBI protein database under accession number NR_029518.1, version GI:262205774.

miR-126 is a standard term well known to those skilled in the art. In particular, the sequence of the human form of miR-126 is given in the NCBI protein database under accession number NC_029695.1, version GI: 262205369.

miR-150 is a standard term well known to those skilled in the art. In particular, the sequence of the human form of miR-150 is given in the NCBI protein database under accession number NC_029703.1, version GI: 262205410.

miR-191 is a standard term well known to those skilled in the art. In particular, the sequence of the human form of miR-191 is given in the NCBI protein database under accession number NC_029690.1, version GI:262205347.

miR-197 is a standard term well known to those skilled in the art. In particular, the sequence of the human form of miR-197 is given in the NCBI protein database under accession number NC_029583.1, version GI: 262206094.

miR-223 is a standard term well known to those skilled in the art. In particular, the sequence of the human form of miR-223 is given in the NCBI protein database under accession number NC_029637.1, version GI: 262206350.

miR-320 is a standard term well known to those skilled in the art. In particular, the sequence of the human form of miR-320 is given in the NCBI protein database under accession number NR_029714.1, version GI: 262205473.

miR-454 is a standard term well known to those skilled in the art. In particular, the sequence of the human form of miR-454 is given in the NCBI protein database under accession number NR_030411.1, version GI:262205121.

miR-486 is a standard term well known to those skilled in the art. In particular, the sequence of the human form of miR-486 is given in the NCBI protein database under accession number NC_030161.1, version GI: 262205146.

RNU6b is a standard term well known to those skilled in the art. In particular, the sequence of the human form of RNU6b is given in the NCBI protein database under accession number NC_002752.1, version GI: 84872030.

For the avoidance of doubt the specific sequences of the markers mentioned above are defined with respect to the version present in the database at the priority date of the present application. The specific sequences of the markers are exemplary. Those skilled in the art will appreciate that polymorphic variants exist in the human population and that the identification of such polymorphic variants is standard practice to those skilled in the art.

There are numerous ways of determining the level of the microRNAs, including Northern blotting, microRNA arrays, real-time RT-PCR methods, next generation sequencing, differential display, RNA interference, RNase protection methods, etc. Such methods are well known to those skilled in the art (see for example Ach et al., BMC Biotechnology, 8, 69, 2008). Preferably the levels of the microRNAs are measured using real-time RT-PCR methods.

In order to determine whether the level of the markers referred to above is greater than (high/increased) or less than (low/reduced) normal, the normal level of a relevant population of non-diabetic individuals is typically determined. The relevant population can be defined based on, for example, diet, lifestyle, age, ethnic background or any other characteristic that can affect the normal levels of the markers. Once the normal levels are known, the measured levels can be compared and the significance of the difference determined using standard statistical methods. If there is a substantial difference between the measured level and the normal level (i.e. a statistically significant difference), then the individual from whom the levels have been measured may be considered to have diabetes or to be at risk of developing diabetes.

The method according to the first aspect of the present invention allows the identification of individuals with type II diabetes as well as the effectiveness of diabetic treatments. The method therefore ensures the correct identification of diabetes and can also be used to monitor the effectiveness of treatments. The method according to the second aspect of the present invention allows the identification of individuals that are likely to develop type II diabetes . This method therefore enables preventative action to be taken, such as changes to the diet and lifestyle of the individual, as well as medical intervention.

According to a third aspect of the present invention there is provided a method of predicting myocardial infarction comprising determining in a sample obtained from an individual the level of at least 2 microRNAs selected from the group consisting of miR-126, miR-223 and miR-197 or selected from the group consisting of miR-126, miR-24 and miR-197.

It has been found that by making the determination set out above it is possible to determine with high specificity and sensitivity whether an individual has or is likely to develop a cardiovascular disorder. Specificity is defined as the proportion of true negatives (individuals that do not have or do not develop a cardiovascular disorder) identified as such in the method. Sensitivity is defined as the proportion of true positives (individuals that have or are likely to develop a cardiovascular disorder) identified as such in the method. The method provides a highly accurate test that can be performed relatively easily using the three biomarkers.

The sample used in the third aspect of the present invention is as defined above. Furthermore, the methods of determining the level of miR-126, miR-223, miR-24 and miR-197, are as defined above.

Preferably, the method according to the third aspect of the present invention comprises determining in a sample obtained from an individual the level of:
i. miR-126 and miR-223; or
ii. miR-126 and miR-24.

Preferably, the level of miR-197 is also determined.

It is further preferred that the method comprises determining in a sample obtained from an individual the level of miR-126 and miR-223. Preferably, the level of miR-197 may also be determined.

The present invention is now described by way of example only with reference to the following figures.

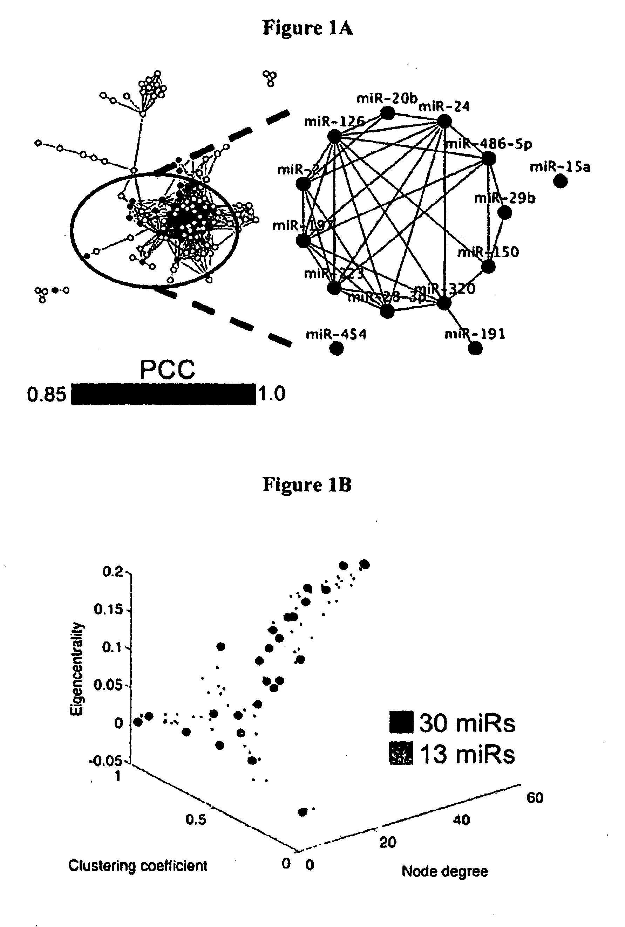

Figure 1 shows a miRNA co-expression network and miRNA topology values. A) A co-expression network consisting of 120 miRNAs and 1020 co-expression links with Pearson correlation coefficient (PCC) values above 0.85. A node represents a miRNA, while an edge represents a presence of co-expression. B) Topological values for 30 miRNAs in the network. Among the 30 differentially expressed miRNAs, 13 occupied topologically critical locations.

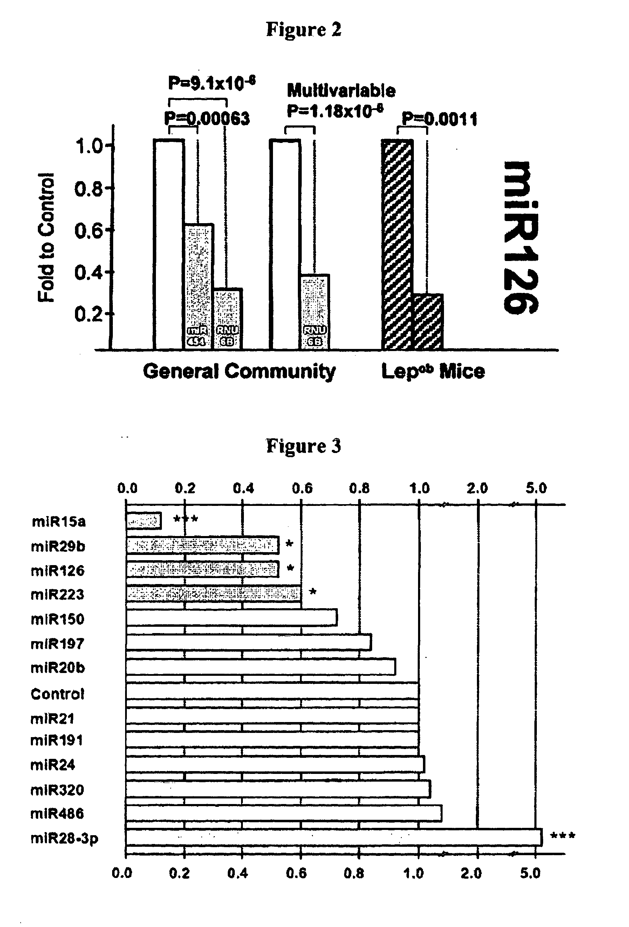

Figure 2 shows the association of plasma miR-126 with diabetes (DM). 13 plasma miRNAs were quantified by qPCR in patients with prevalent DM and matched controls (data not shown). Endothelial miR-126 is shown as an example. Fold-changes and p-values are derived from univariate and multivariate analyses. Bars on the right provide a comparison with fold-changes observed in plasma of hyperglycaemic Lep^{ob} mice.
Figure 3 shows the association of plasma miRNAs with incident DM. 13 plasma miRNAs were quantified by qPCR in patients who developed DM over a 10-year observation period and matched controls. * denotes p<0.05, *** p<0.001.

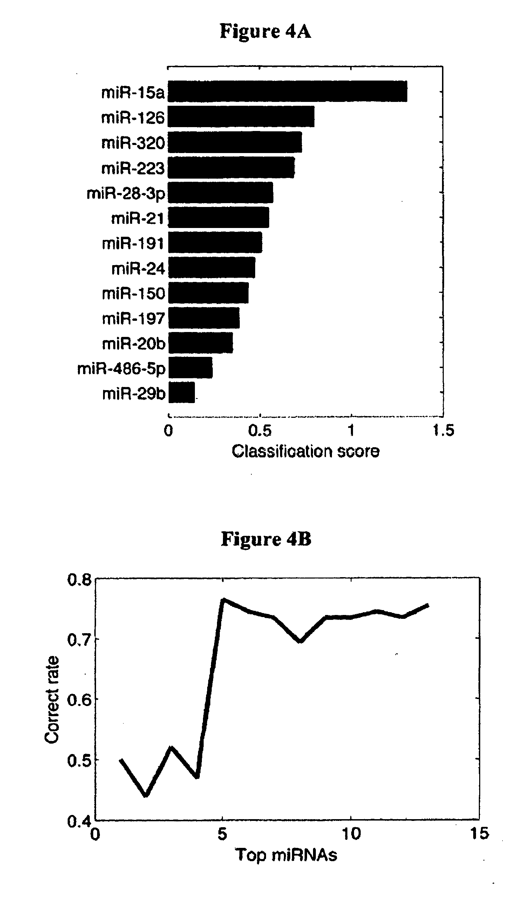

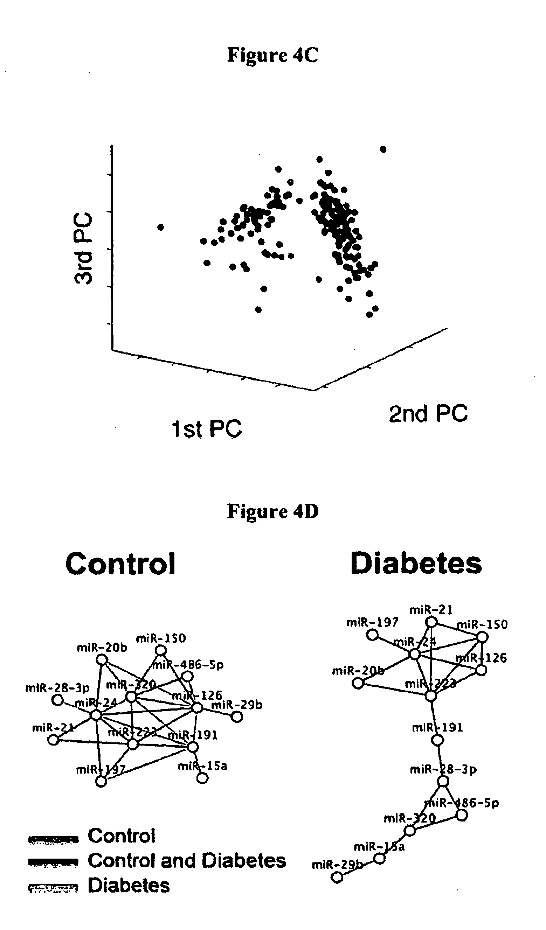

Figure 4 shows the principal component analysis (PCA) and network properties. **A)** Classification efficiency of 13 miRNAs across controls (n=99), incident DM (n=19), and prevalent DM (n=80). Higher feature importance score is indicative of a greater classification potential. **B)** Classification accuracy of support vector machine (SVM) classifier applied to an aggregation of top N-scoring miRNAs. Best classification accuracy (0.77%) was achieved using top 5-scoring miRNAs. Aggregation of additional miRNAs did not increase SVM performance. **C)** PCA decomposition of the top 5 scoring miRNAs was sufficient to discriminate 91/99 (92%) controls and 56/80 (70%) patients with DM. Notably, 10/19 (52%) subjects with incident DM were already clustered with DM patients before the manifestation of the disease. **D)** Differential network structure between 13 miRNAs in controls (n=91) and patients with prevalent DM (n=56) as inferred by context likelihood relatedness algorithm. Control (19 nodes, 23 links) and DM (13 nodes, 18 links) networks shared 10 miRNAs and 11 links. Nonetheless, the disease state was characterized by substantial edge rewiring as defined by changed miRNA-miRNA co-expression values (miR-15a).

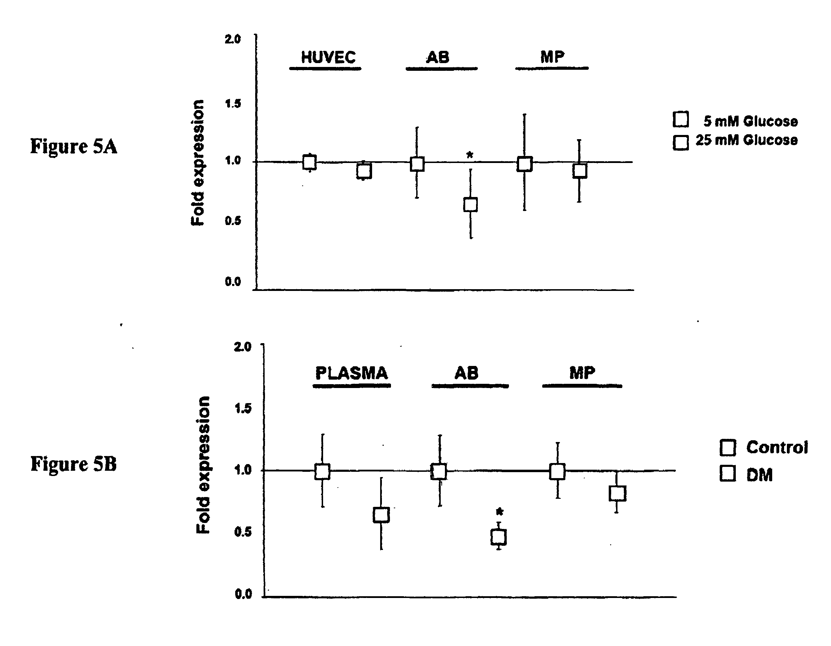

Figure 5 shows miR-126 is reduced in endothelial derived apoptotic bodies. **A)** miR-126 levels in endothelial cells (HUVEC), endothelial-derived apoptotic bodies (AB) and microparticles (MP) in response to high glucose. **B)** miR-126 levels in circulating apoptotic bodies (AB) and microparticles (MP) isolated from plasma samples of patients with DM and controls. Values represent fold changes ± SE, * P<0.05.

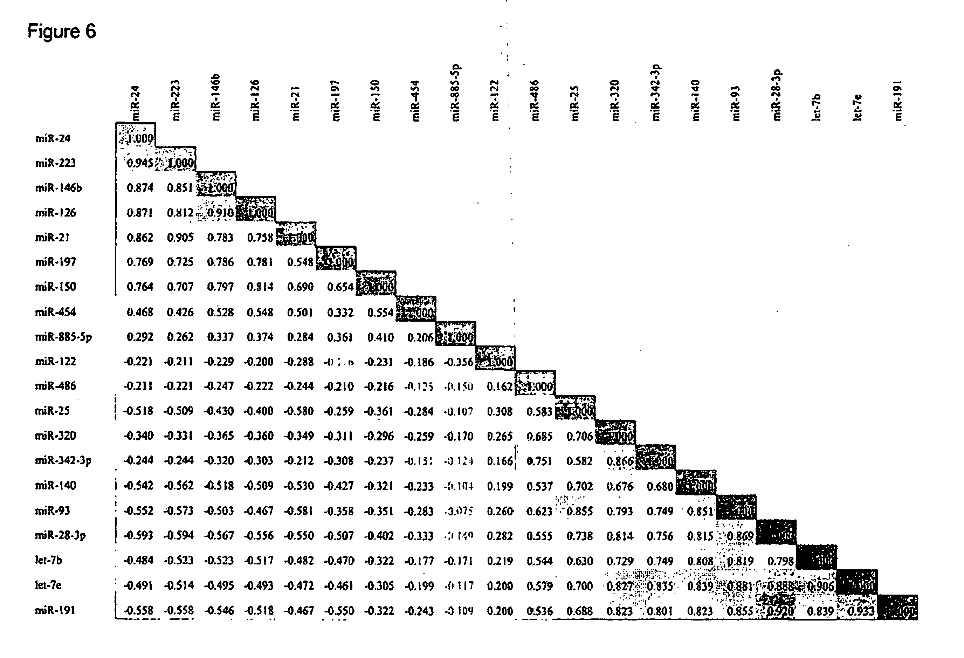

Figure 6 shows the correlation between various miRs in the individual studied.

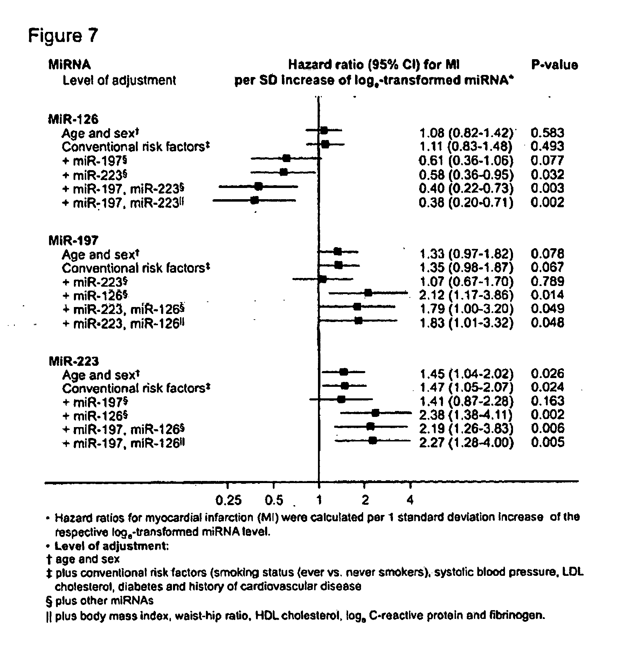

Figure 7 shows the plasma miRNA signature for incident myocardial infarction (MI). The graph shows risk estimates for the three miRNAs most consistently associated with disease risk (as identified by AIC-based models and the technique of least absolute shrinkage and selection operator). Hazard ratios (95% CI) were derived from standard Cox regression models with progressive levels of adjustment. MiR-223 and miR-24 are highly correlated and reach almost perfect correlation (r = 0.945). Hence, exchanging miR-223 for miR-24 gives similar results.

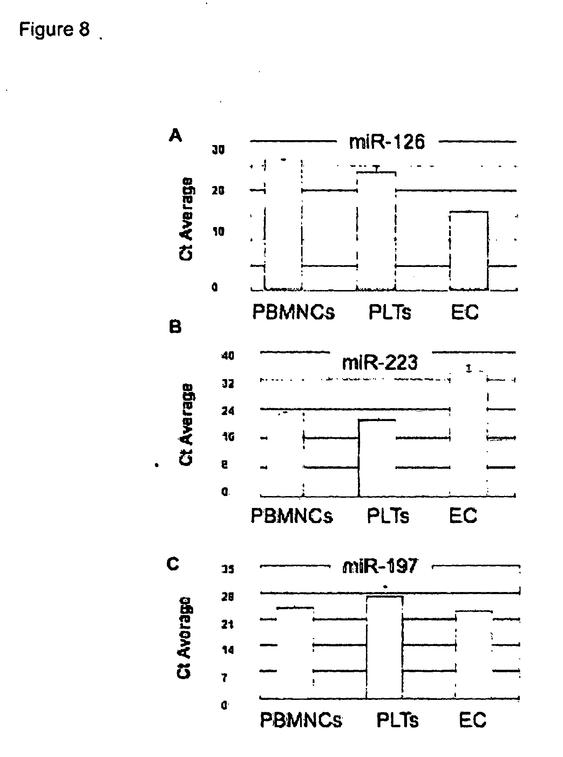

Figure 8 shows MiRNA levels in different cell types. Assessment of miR-126 (A), miR-223 (B) and miR-197 (C) expression in peripheral blood mononuclear cells (PBMCs), platelets (PLTs) and endothelial cells (EC) was performed using quantitative polymerase chain reaction. Cycle threshold (Ct) values are provided. The data shown are means ± SD derived from 3 different preparations.

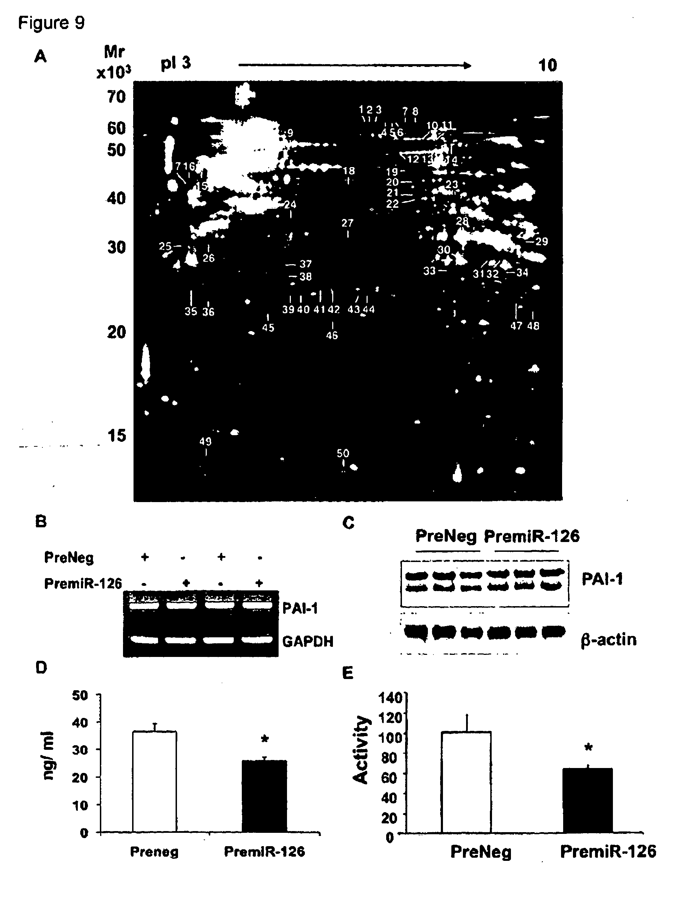

Figure 9 shows the difference in-gel electrophoresis (DIGE). DIGE analysis of human umbilical vein endothelial cells 48 hours after treatment with scrambled miRNAs or miR-126 precursors **(A).** Deregulated proteins are numbered and listed in Table 3. MiR-126 does not affect PAI-1 mRNA levels **(B)** and net expression **(C)** but regulates PAI-1 protein secretion **(D)** and PAI activity **(E)** of the conditioned medium as assessed by ELISA. The data presented are means **±** SD for 3 independent experiments. *P-value for difference versus PreNeg controls < 0.05.

### EXAMPLES

### Example 1

### Materials and Methods

### Study Subjects.

The Bruneck Study is a prospective population-based survey initially designed to investigate the epidemiology and pathogenesis of atherosclerosis and later extended to study all major human diseases including diabetes ^{17-19,31,32}. At the baseline evaluation in 1990, the study population was recruited as a sex- and age-stratified random sample of all inhabitants of Bruneck (Bolzano Province, Italy) 40 to 79 years old (125 women and 125 men in the fifth to eighth decades each). A total of 93.6% participated, with data assessment completed in 919 subjects. During 1990 and the reevaluation in 1995 (the first five-year period), a subgroup of 63 individuals died or moved away. In the remaining population follow-up was 96.5% complete (n=822). RNA extraction was performed from blood specimens collected as part of the 1995 follow-up in 822 individuals. Follow-up in 2000 and 2005 was 100% complete for clinical endpoints and > 90% complete for repeated laboratory examinations. The protocols of the Bruneck study were approved by the appropriate ethics committees, and all study subjects gave their written informed consent before entering the study.

### Clinical History and Examination.

Smoking status was assessed in each subject. Regular alcohol consumption was quantified in terms of grams per day. Hypertension was defined as blood pressure (mean of 3 measurements) ≥ 140/90 mm Hg or the use of antihypertensive drugs. Body mass index was calculated as weight divided by height squared (kg/m2). Waist and hip circumferences (to the nearest 0.5 cm) were measured by a plastic tape meter at the level of the umbilicus and of the greater trochanters, respectively, and waist-to-hip ratios (WHR) were calculated. Socioeconomic status was assessed on a three-category scale (low, medium, high) based on information about occupational status and educational level of the person with the highest income in the household. Family history of DM refers to first-degree relatives. Physical activity was quantified by the Baecke Score (index for sports activity)³³.

### Laboratory Methods.

Blood samples were drawn after an overnight fast and 12 hours' abstinence from smoking. A 75 g oral glucose load (OGTT) was administered to all subjects without known DM and blood samples were collected after 120 minutes in order to establish glucose tolerance. High-density lipoprotein (HDL) cholesterol was determined enzymatically (CHOD-PAP method, Merck; CV 2.2% to 2.4%). Low-density lipoprotein (LDL) cholesterol was calculated with the Friedewald formula except in subjects with triglycerides >4.52 mmol/L in whom it was directly measured. Markers of inflammation and all other laboratory parameters were assessed by standard methods as detailed previously ^{17-19,31,32}.

### Ascertainment of Diabetes.

In 1995, presence of DM was established according to World Health Organization criteria ¹⁸, i.e. when fasting glucose was ≥ 7 mmol/l (126 mg/dl) or when 2-h OGTT glucose was ≥ 11.1 mmol/l (200 mg/dl), or when the subjects had a clinical diagnosis of the disease. During the subsequent 10-year follow up period incident cases of DM were established by the same criteria except for 2-h glucose levels which were not available in 2000 and 2005. Self-reported DM status was obligatorily confirmed by reviewing the medical records of the subject's general practitioners and files of the Bruneck Hospital. No self-reported case of DM was accepted without a validated confirmation.

### Obese mice.

All animal experiments were performed according to protocols approved by the Institutional Committee for Use and Care of Laboratory Animals. To determine whether miRNA profiles identified in diabetic subjects also apply to hyperglycaemic animals, the inventors quantified plasma levels in obese mice. For this purpose 8-12 week old Lep_{ob} mice (previously known as ob/ob) (n=6) were purchased from Jackson laboratories. C576L/6J mice were used as a control (n=6). A total of 1 ml of blood was harvested from each mouse and plasma was isolated following centrifugation at 1,200g for 20 min at 4°C. Plasma samples were aliquoted and stored at -80°C.

### RNA isolation from plasma.

Total RNA was prepared using the miRNeasy kit (Qiagen) according to the manufacturer's recommendations. In brief, 200 µl of plasma was transferred to an Eppendorf tube and mixed thoroughly with 700µl of QIAzol reagent. Following a brief incubation at ambient temperature, 140 µl of chloroform were added and the solution was mixed vigorously. The samples were then centrifuged at 12,000 rpm for 15 min at 4°C. The upper aqueous phase was carefully transferred to a new tube and 1.5 volumes of ethanol were added. The samples were then applied directly to columns and washed according to the company's protocol. Total RNA was eluted in 25 µl of nuclease free H₂O. A fixed volume of 3 µl of RNA solution from the 25 µl eluate was used as input in each reverse transcription reaction.

### RNA isolation from circulating apoptotic bodies and microparticles.

To isolate circulating microvesicles, plasma samples from diabetic or healthy subjects were pooled together (30 samples per pool) and three pools were generated per group. Microvesicles were isolated by ultracentrifugation. In brief, plasma samples were centrifuged for 10 min at 800g to remove any precipitate and the apoptotic bodies were then isolated by centrifugation of the supernatant at 10,600 rpm for 20 min. The pellet was resuspended in PBS. An additional centrifugation of the supernatant at 20,500 rpm for 2h was performed to isolate microparticles. The pelleted microparticles were resuspended in PBS and the remaining supernatant was considered as microvesicle-depleted plasma. Total RNA was isolated using the miRNeasy kit as described above.

### Reverse Transcription and Preamplification.

To assess levels of specific miRNAs in individual plasma samples a fixed volume of 3 µl of RNA solution from the 25 µl eluate was used as input in each reverse transcription (RT) reaction. An RT reaction and pre-amplification step were set up according to the company's recommendations and performed as described above. RT-PCR and pre-amplification products were stored at -20°C. miRNAs were reverse transcribed using the Megaplex Primer Pools (Human Pools A v2.1 and B v2.0) from Applied Biosystems. Pool A enables quantitation of 377 human miRNAs while an additional 290 miRNAs were assessed using Pool B. In each array, three endogenous controls and a negative control were included for data normalization. RT reaction was performed according to the company's recommendations (0.8 µl of Pooled Primers were combined with 0.2 µl of 100mM dNTPs with dTTP, 0.8 µl of 10x Reverse-Transcription Buffer, 0.9 µl of MgCl₂ (25mM), 1.5 µl of Multiscribe Reverse-Transcriptase and 0.1 µl of RNAsin (20U/µl) to a final volume of 7.5 µl. The RT-PCR reaction was set as follows: 16°C for 2 min, 42°C for 1 min and 50°C for 1 sec for 40 cycles and then incubation at 85°C for 5 min using a Veriti thermocycler (Applied Biosystems). The RT reaction products were further amplified using the Megaplex PreAmp Primers (Primers A v2.1 and B v2.0). A 2.5 µl aliquot of the RT product was combined with 12.5 µl of Preamplification Mastermix (2x) and 2.5 µl of Megaplex PreAmp Primers (10x) to a final volume of 25 µl. The pre-amplification reaction was performed by heating the samples at 95°C for 10 min, followed by 12 cycles of 95°C for 15 sec and 60°C for 4 min. Finally, samples were heated at 95°C for 10 min to ensure enzyme inactivation. Pre-amplification reaction products were diluted to a final volume of 100 µl and stored at -20°C.

### Taqman miRNA Array.

The expression profile of miRNAs in plasma samples was determined using the Human Taqman miRNA Arrays A and B (Applied Biosystems). PCR reactions were performed using 450 µl of the Taqman Universal PCR Master Mix No AmpErase UNG (2x) and 9 µl of the diluted pre-amplifcation product to a final volume of 900 µl. 100µl of the PCR mix was dispensed to each port of the Taqman miRNA Array. The fluidic card was then centrifuged and mechanically sealed. QPCR was carried out on an Applied Biosystems 7900HT thermocycler using the manufacturer's recommended programme. Detailed analysis of the results was performed using the Real-Time Statminer Software (Integromics).

### Taqman qPCR Assay.

Taqman miRNA assays were used to assess the expression of individual miRNAs. 0.5µl of the diluted pre-amplification product were combined with 0.25 µl of Taqman miRNA Assay (20x) (Applied Biosystems) and 2.5 µl of the Taqman Universal PCR Master Mix No AmpErase UNG (2x) to a final volume of 5 µl. QPCR was performed on an Applied Biosystems 7900HT thermocycler at 95°C for 10 min, followed by 40 cycles of 95°C for 15 sec and 60°C for 1 min. All samples were run in duplicates and standardized to miR-454 and RNU6b using SDS2.2 (Applied Biosystems) software. For sensitivity analyses, levels of miRNAs for PCR efficacy were corrected using the LinRegPCR software. ¹⁷

### Endothelial cell culture and isolation of microvesicles.

Human umbilical vein endothelial cells (HUVECs) were purchased from Cambrex and cultured on gelatin coated flasks in M199 medium supplemented with 1 ng/ml endothelial cell growth factor (Sigma), 3 µg/ml endothelial growth supplement from bovine neural tissue (Sigma), 10 U/ml heparin, 1.25 µg/ml thymidine, 5% foetal bovine serum, 100 µg/ml penicillin and streptomycin. HUVECs exposed to high glucose (25mM) were cultured in complete medium for 6 days. As a control (5mM glucose), HUVECs were cultured in complete medium supplemented with mannitol, to exclude effects of osmotic stress. On day 5, cells were counted and equal numbers of cells was seeded on T75 flasks and incubated for an additional day. Subsequently, the cells were deprived of serum and growth factors for 24 h and apoptotic bodies and microparticles were isolated as described previously ²⁹. In brief, the conditioned medium was harvested and centrifuged for 10 min at 800 g to remove the cell debris while cells were lysed in QIAzol reagent (1 ml per T75 flask). The lysates were stored at -20°C for miRNA expression studies. Apoptotic bodies were then isolated by centrifugation of the supernatant at 10,600 rpm for 20 min. The pellet was resuspended in PBS (50µl of PBS per T75) and stored at -80°C. An additional centrifugation of the supernatant at 20,500 rpm for 2h was performed to isolate the microparticles released by the endothelial cells. Microparticles were also resuspended in PBS (500µl of PBS per T75) and stored at -80°C. Total RNA was isolated from 200µl of apoptotic bodies in PBS using the miRNeasy kit as described above. Total RNA was eluted in 25 µl of nuclease free H₂O. RNA was quantified using the NanoDrop spectrophotometer and 20 ng of total RNA were used for reverse transcription.

### miRNA co-expression network inference and analysis.

Similarity in miRNA expression profiles was interrogated using either Pearson correlation coefficients (PCC) or context likelihood of relatedness (CLR) between all possible miRNA pairs ³⁴. Pairs that maintained dependence above a predefined threshold were represented in the form of an undirected weighted network, where nodes correspond to miRNAs and links (edges). While PCC is a way to measure linear relationships between features (miRNAs), CLR relies on a mutual information metric and does not assume linearity ³⁶⁻³⁷, thus possessing some flexibility to detect biological relationships that may otherwise be missed. PCC was used to detect clusters of similarly expressed miRNAs from a high throughput space of expression arrays, while CLR was used to identify all non-randomly associated qPCR-validated miRNA profiles. While PCC and CLR sometimes yield similar results, CLR was chosen for validated miRNAs because it is more sensitive to non-linear dynamics of miRNA expression than PCC and significantly outperforms other network inference methods (e.g. ARACNE) in identifying biologically meaningful relationships ^{20,38}. The PCC threshold was set to a point where miRNA co-expression network began to acquire a scale-free architecture, which is a characteristic of most real-world networks, including, biological ³⁹. The CLR threshold was chosen such that all 13 miRNAs could be represented in the network while retaining the smallest possible number of links between them

### Topological analysis.

During pre-screening, miRNAs were studied by virtue of their topology in the global miRNA co-expression network as well as individual over- or under-expression. For each miRNA topological parameters including node degree, clustering coefficient, and eigenvector centrality were systematically calculated. Node degree is defined as the total number of edges that are connected to a given miRNA. Clustering coefficient is the degree to which miRNAs tend to cluster together. Eigenvector centrality is a measure of miRNA importance, such that a particular miRNA receives a greater value if it is strongly correlated with other miRNAs that are themselves central within the network.

### Network clustering.

Modular structure of the miRNA co-expression network was identified using the Markov Clustering (MCL) algorithm ²¹. This is an efficient, unsupervised, and highly accurate graph clustering approach based on graph flow simulation ⁴⁰. A distinct advantage of MCL is its ability to avoid incorrect clustering assignments in the presence of false negative edges. This is due to the fact that MCL discovers clusters by virtue of miRNAs sharing higher-order connectivity in their local neighborhood and not merely pair-wise linkages.

### Feature selection, principal component analysis, and classification.

Predictive significance of validated miRNAs was calculated using bootstrap aggregation for an ensemble of 10000 decision trees. Each miRNA profile was assigned a feature importance value based on how well it can differentiate Control, Incident Diabetes, and Prevalent Diabetes classes. Principal component analysis (PCA), a technique that reduces the data to two or more uncorrelated components that explain most of the variance in the data, was then used to determine whether Controls, Incident Diabetes, and Prevalent Diabetes cohorts could be distinguished. The discriminatory power of the PCA was quantified using the Support Vector Machines (SVM) algorithm, a supervised learning method. SVM were trained on half of the data selected at random and validated on the remaining samples. This procedure was repeated 10 times and the final correct classification rate was presented as an average of all SVM iterations. Classification is not limited to SVM and may be carried out with comparable accuracy using other machine learning approaches familiar to those skilled in the art.

### Sampling strategy and statistical analysis.

For the initial micro-array screening, 2 pools of plasma obtained from 5 individuals with DM each (randomly selected from the 80 patients with diabetes in the Bruneck study) and 6 pools of plasma obtained from controls identical in age, sex, risk factor profile (LDL, smoking, hypertension) and atherosclerosis status were used. qPCR of 30 candidate miRNAs identified by microarray and miRNA co-expression network inference was performed in 2 groups of 15 samples each comprised of diabetic patients and their age and sex matched healthy controls. qPCR in a larger cohort was performed for the 13 miRNAs that showed correlation with DM. Taqman assays were run in duplicate in samples of (a) all subjects with diabetes in the Bruneck cohort (1995, n=80), (b) 19 patients who developed DM between 1995 and 2005 and (c) in 80 and 19 matched controls identical for age and sex with a fasting glucose levels below 6.1 mmol/L (110 mg/dL), 2-hr glucose below 7.7 mmol/L (140 mg/dL) and no history of DM. In the case of several suitable matches all were numbered in ascending order and one was selected by means of a computer-based random number generator. Finally, miR-126 was measured in the entire population of 822 individuals. For the lack of generally accepted standards all qPCR data were analyzed as unadjusted Ct-values and standardized to both miR-454 and RNU6b, a small nuclear RNA, which fulfilled the following criteria: detectable in all samples, low dispersion of expression levels and null-association with DM status. Moreover, the miR-454 expression profile showed little association with other miRNAs and positioned outside the co-expression modules of the complex miRNA network in plasma (Figure 1). Data were analysed using SPSS version 15.0 and STATA version 10 software packages. Continuous variables were presented as mean±SD or median (interquartile range), and dichotomous variables as numbers and percentages. Fold changes of individual miRNAs were calculated for each pair of matched (pre)diabetic cases and controls by dividing the standardized expression levels of the miRNAs. The median of fold changes is presented in the figures. Differences in miRNA levels between subjects with prevalent or incident diabetes and corresponding groups of matched controls were analyzed using the non-parametric Wilcoxon test for related samples with computation of exact P values. To account for the potential confounding effects of life-style features and other variables related to DM and to analyze for interactions the inventors additionally performed logistic regression analyses for matched data that include logₑtransformed expression levels of miRNAs (one per model) and the following variables: social status, family history of DM, body mass index, waist-to-hip ratio, smoking status, alcohol consumption (g/day), physical activity (sports index) and high-sensitivity C-reactive protein. Details on model construction were described by Hosmer and Lemeshow ³⁵. First-order interactions between miRNAs and the above variables as well as age and sex were calculated by inclusion of appropriate interaction terms. None of these terms achieved statistical significance. All P values presented are two-sided.

### Results

### Comprehensive miRNA profiling.

For the initial screening, Human Taqman miRNA arrays (CardA v2.1 and CardB v2.0, Applied Biosystems) covering 754 small non-coding RNAs were applied to 8 pooled samples, 2 consisting of diabetic subjects and 6 of appropriate controls (see methods). Of the 148 miRNAs with Ct-values < 36, 130 miRNAs were detected using fluidic Card A and therefore all further analysis focused on this dataset resulting in the identification of 30 differentially expressed plasma miRNAs in patients with DM (data not shown).

### miRNA network analysis.

The 30 differentially expressed miRNAs were sampled by virtue of their localization in the miRNA co-expression network as marker selection using network topology is more reproducible than assessment of individual over- or under-expression ²². The miRNA co-expression network was dominated by a small number of hubs that linked with many loosely connected nodes - a property of many biological networks. The miRNA network consisted of 120 miRNAs (nodes) and 1020 co-expression links (edges) (data not shown). Within the network, the 30 differentially expressed miRNAs were topologically central (**Figure 1A**). Thus, it was hypothesized that changes in expression and differential centrality may be indicative of biological importance. As it is yet unclear whether disease-associated nodes are central or peripheral to a biological network, the inventors selected 13 of the 30 differentially expressed miRNAs that displayed extreme spectra of node degrees, clustering coefficients, and eigenvector centrality values (**Figure 1B**). MiR-454 was the only miRNA that showed no association with the expression of other miRNAs and was positioned outside all network modules (**Figure 1A****).** Thus, it was used as an additional normalization control.

### Validation by qPCR.

The 13 topologically unique miRNAs were further quantified by qPCR. All patients with manifest DM at the time of the 1995 evaluation (n=80) were compared to age- and sex-matched controls. Plasma levels of miR-24, miR-21, miR-20b, miR-15a, miR-126, miR-191, miR-197, miR-223, miR-320, miR-486, miR-150 and miR-29b were lower in diabetic subjects while miR-28-3p was generally higher (data not shown). Nine miRNAs standardized to RNU6b showed significant differences between patients with DM and controls and four remained significant after accounting for the multiple comparisons performed (Bonferroni P value <0.000133), including endothelial miR-126 (**Figure 2**). Bonferroni correction, however, is overly conservative in this setting because individual miRNAs are not independent of each other but extensively correlated. In multivariate analyses, all miRNAs except miR-29b were significantly associated with DM - 11 showed an inverse relationship and miR-28-3p a positive one (data not shown). In another run, miR-126 was quantified in the entire Bruneck cohort (n=822) and standardized against miR-454. In logistic regression analyses, miR-126 emerged again as a significant predictor of prevalent diabetes (odds ratio [95%CI], 0.38 [0.26-0.55]; P=2.72x10⁻⁷) and this association persisted in a multivariable approach. As expected, levels of miRNAs were inversely correlated with fasting glucose levels in both patients with DM and control subjects (r∼-0.191 to -0.335, p<0.05 each except miR-29b and miR-320) and less consistently with 2-hr glucose levels (r = -0.091 to -0.239) and Hba1c concentrations (r = -0.093 to -0.312). Most of the miRNA changes observed in DM could independently be replicated in plasma samples of 8-12 week old hyperglycaemic Lep^{ob} mice (**Figure 2**).

### Incident diabetes and miRNA networks.

Importantly, several miRNAs were already altered prior to manifestation of DM. A total of 19 subjects, who were normoglycaemic in 1995, developed DM over the 10-year follow-up period (1995-2005 mean interval until diagnosis of diabetes 79.2 months). Baseline levels of miR-15a, miR-29b, miR-126 and miR-223 were significantly lower in these subjects while miR-28-3p was higher compared to matched controls (**Figure 3**).

### MiRNAs as biomarkers in diabetes.

To determine whether miRNAs can correctly distinguish individuals with prevalent or incident diabetes from healthy controls, the inventors evaluated the predictive power of miRNAs by performing a principal component analysis (PCA). Interestingly, when the 5 highest scoring miRNAs (miR-15a, miR-126, miR-320, miR-223, miR-28-3p) were reduced to 2 principal components, support vector machines (SVM) correctly classified 91/99 (92%) controls and 56/80 (70%) DM cases (**Figure 4**). The 24 DM cases that were classified as normal subjects, had significantly lower levels of fasting glucose (mean±SD, 120.0±28.6 mg/dL vs 147.2±55.0 mg/dL, p=0.005) and Hbalc (mean±SD, 6.03±0.75 % vs 6.66±1.54 %, p=0.016) and represent a selection of well-treated patients with diabetes. Of note, 10/19 (52%) subjects with incident DM were already classified as diabetic before the manifestation of disease. Inclusion of additional miRNAs into the classification did not improve sensitivity or specificity of the model (**Figure 4B**). Thus, these 5 miRNA can be considered the minimal requirement for subject differentiation based on a miRNA signature. Further support to the putative value of miRNAs as diagnostic tools in DM was provided by the inference of miRNA network using context likelihood of relatedness. Topologically, the control network was more interconnected than the DM network (average node degree = 3.9 vs. 2.8 respectively) and contained more central nodes (average eigenvector centrality = 0.24 vs. 0.20 respectively) (**Figure 4**). However, studies in large collectives of patients with DM and pre-diabetes are required to assess the potential of this miRNA signature.
MiRNA-126 in endothelial-derived apoptotic bodies and human vascular disease.
Among the miRNAs most consistently associated with DM, was the endothelial-specific miR-126. This miRNA has previously been shown to govern the maintenance of vascular integrity, angiogenesis, and wound repair ²³⁻²⁴. MiR-126 is released from endothelial cells in microvesicles. To determine whether hyperglycaemia affects miR-126 release from endothelial cells, miRNA levels of HUVECs apoptotic bodies and microparticles derived under normal (5 mM) and high (25 mM) glucose concentrations were compared. While cellular miRNA concentrations remained unaltered, high glucose significantly reduced the miR-126 content in endothelial apoptotic bodies (**Figure 5A**) whereas shedding of other miRNAs except miR-24 was not affected (data not shown). Consistent with the *in vitro* experiments, the reduction in miR-126 levels in patients with DM was confined to circulating apoptotic bodies (**Figure 5B****).**

Finally, evidence from population cohort suggests that loss of miR-126 in plasma correlates with subclinical and manifest peripheral artery disease. In detail, miR-126 was associated with a low ankle-brachial index (<0.9, n=77) (unadjusted and age-/sex-adjusted odds ratio [95%CI] for a 1-SD unit decrease of logₑ-transformed expression level of miR-126, 0.51 [0.39-0.67] P<0.001 and 0.72 [0.54-0.95] P=0.025) and with new-onset symptomatic peripheral artery disease (1995-2005, n=15) (unadjusted and age-/sex-adjusted hazard ratio [95%CI] for a 1-SD unit decrease of logₑ-transformed expression level of miR-126, 0.38 [0.20-0.72] P=0.0032 and 0.46 [0.23-0.93] P=0.030). In the latter analysis 37 subjects with symptomatic peripheral artery disease at baseline were excluded.

### Discussion

In this study, the inventors demonstrate the existence of a distinct plasma miRNA signature in patients with DM including a significant reduction of miR-126 and provide preliminary evidence for a potential prognostic value of miRNAs in this setting. They also demonstrate that the loss of miR-126 in plasma correlates with subclinical and manifest peripheral artery disease.

### Plasma miRNAs in DM.

Plasma miRNAs are packaged in membranous microvesicles that protect them from RNase degradation. These microvesicles can be released by a variety of cell types and change in numbers, cellular origin and composition depending on the disease state ²⁵. Accumulating evidence support the notion that microvesicles are not just by-products resulting from cell activation or apoptosis. Instead, they constitute a novel type of cell-cell mechanism of communication. Our assessment of 13 miRNAs in patients with DM and their age- and sex-matched controls revealed a distinct pattern of plasma miRNAs that form a tightly interconnected network (**Figure 1** and **4**). Of particular note is the observation that the deregulation of several plasma miRNAs antedated the manifestation of DM (**Figure 3**). The clinical relevance of these findings is that (1) miRNAs are assumed to be crucially involved in the epigenetic regulation of key metabolic pathways in DM and may provide novel insights into the pathophysiology and complications of disease (in this respect, endothelial miR-126 deserves special consideration); (2) miRNA profiles can serve as biomarkers enabling early disease prediction and intervention, even in a pre-diabetic stage.

### MiRNA profiles as biomarkers.

Using differential expression and concepts of network topology, the inventors identified and assessed the expression of 13 miRNAs. PCA of the 13 studied miRNAs indicates that 5 miRNAs (miR-15a, miR-126, miR-320, miR-223, miR-28-3p) with the highest scores are necessary and sufficient for a non-redundant classification. Importantly, the miRNA network was significantly affected by DM. Inferred DM network was differentially wired compared to the control network, an observation that is consistent with a recent report of miRNA rewiring in patients with leukemia compared to healthy controls ²⁶. Topologically, the control network was more robust against hub removal than the DM network (data not shown), suggesting a "protective" topology under normal conditions²⁷.

### MiR-126 and diabetes.

Whereas most plasma miRNAs are widely expressed, miR-126 is considered endothelial-specific and ranks among the miRNAs most consistently affected by DM. Systemic endothelial dysfunction is a known consequence of DM and results in vascular complications and abnormal angiogenic response. MiR-126 has been shown to play a pivotal role in maintaining vascular integrity ²³⁻²⁴ and the release of miR-126 in apoptotic bodies confers vascular protection in a paracrine manner ²⁸. The present work expands on these findings by demonstrating that high glucose concentrations reduce miR-126 levels in endothelial apoptotic bodies without altering the cellular miR-126 content. Notably, the observed reduction of miR-126 in plasma of patients with DM was also confined to circulating apoptotic bodies. MiR-126 has also herein been shown to be an effective marker of vascular disorders, including peripheral artery disease.

### Example 2

### Materials and Methods

### Study subjects.

The same subjects as used for Example 1 above were studied. Fatal and non-fatal myocardial infarction (MI) was ascertained following the WHO criteria for definite disease status.

### RNA isolation from plasma.

Total RNA extraction from plasma was performed using the miRNeasy kit (Qiagen) as described previously above for Example 1.

### Reverse transcription and pre-amplification and Taqman qPCR assay.

Performed as described above for Example 1.

### Peripheral blood mononuclear cells (PBMCs) and platelets (PLTs).

PBMCs were isolated according to standard protocol. Heparinized whole blood (5 to 8 mL) was diluted to 10 mL with phosphate-buffered saline (PBS) (pH 7.4), layered on top of 5 mL Histopaque 1083, and centrifuged for 30 minutes at 400g. PBMCs were washed twice, resuspended in PBS, and counted with a haemocytometer. Platelets (PLTs) were isolated from healthy subjects as previously described.²⁹ In brief, blood was drawn using acid citrate dextrose as anticoagulant (ACD: 120 mM sodium citrate, 110 mM glucose, 80 mM citric acid, 1:7 vol/vol) and centrifuged for 17 minutes at 200g and 30°C in the presence of indomethacin (10 µM; Sigma-Aldrich). The platelet-rich plasma was then centrifuged for another 10 minutes at 1000g in the presence of prostacyclin (0.1 µg/mL; Sigma-Aldrich). The resulting platelets were resuspended in modified Tyrode-HEPES buffer (145 mM NaCl, 2.9 mM KCl, 10 mM HEPES, 1 mM MgCl₂, 5 mM glucose, pH 7.3) at a concentration of 4 x 10⁸/mL.

### Endothelial cell culture

Human umbilical vein endothelial cells (HUVECs) were purchased from Cambrex and cultured on gelatin-coated flasks in M199 medium supplemented with 1 ng/mL endothelial cell growth factor (Sigma), 3 µg/mL endothelial growth supplement from bovine neural tissue (Sigma), 10 U/mL heparin, 1.25 µg/mL thymidine, 10% foetal bovine serum, 100 µg/mL penicillin and streptomycin. The cells were subcultured every 3 days to a ratio 1:4.

### Cell transfection and proteomics analysis

For miR-126 overexpression, HUVECs were transfected using Lipofectamine RNAiMAX (Invitrogen) according to the company's recommendations. In brief, cells were plated on a T75 flask and the following morning transfected in serum free medium using premiR-126 or the negative control preNeg (Ambion) to a final concentration of 90nM. Cells were harvested 48 hours post transfection for proteomics analysis. Key techniques involved adaptations of previously published protocols, including those for difference in-gel electrophoresis and liquid chromatography tandem mass spectrometry (LC-MS/MS).

### Reverse transcriptase-PCR

Total RNA was converted to cDNA using the Promega Reverse Transcription System (Promega, Madison WI). cDNA products were amplified by PCR using gene-specific primers. The primers used were PAI-1 forward: CAA CTT GCT TGG GAA AGG AG, and PAI-1 Reverse: GGG CGT GGT GAA CTC AGT AT, GAPDH forward: CGG AGT CAA CGG ATT TGG TCG TAT; and GAPDH reverse: AGC CTT CTC CAT GGT GGT GAA GAC. PCR conditions were as follows: 94°C for 3 min and then 30 cycles for PAI-1 or 28 cycles for GAPDH at 94°C for 30 s, 58°C for 1 min and 72°C for 1 min, followed by 72°C for 10 min. PCR products were separated by agarose gel electrophoresis.

### Immunoblotting

HUVECs were washed twice with cold PBS (4°C, pH 7.4), harvested on ice in RIPA buffer (10 mM Tris (pH 8.0), 10 mM EDTA, 140 mM NaCl, 1% Triton, 1% Na deoxycholate, 0.1% SDS, and 25 mM β-glycerol-PO₄, supplemented with 1 µg/mL leupeptin, I µg/mL aprotinin, and 100 µM phenylmethylsulfonyl fluoride), and then centrifuged at 13000 rpm at 4°C for 15 min. The supernatant was harvested and used for Western blot analysis. For immunoblotting, 20 µg of cell lysate was applied to an SDS-PAGE. Antibodies against PAI-1 and β-action were from Abcam. Specific antibody-antigen complexes were detected by using the ECL Western Blot Detection Kit (Amersham Pharmacia Biotech UK).

### Plasminogen activator inhibitor (PAI-1) assays

Conditioned media of HUVECs transfected with premiR-126 or the negative control preNeg were harvested 24 hours post transfection. PAI activity was assessed with the PAI activity kit from Millipore according to the company's recommendations. In brief, the PAI in the conditioned media was activated following incubation with activation buffer (4M Guanidine HCl, 20mM Sodium Acetate, pH 5.6, 200 mM NaCl, 0.1% Tween 20). Subsequently, an enzymatic reaction was set up using urokinase-type plasminogen activator (uPA) as a positive control. In this reaction a chromogenic substrate was cleaved by active uPA and detected by its optical density (OD) at 405nm. Addition of PAI sample blocked the cleavage of substrate by uPA. Enzymatic activity was estimated by comparing the absorbance values to a standard curve.

### Statistical analysis

Data were analyzed using the statistical packages SPSS version 15.0 and STATA version 10.1. Normally distributed continuous variables were presented as means ± standard deviation (SD), variables with a skewed distribution as geometric means ± geometric SD, and dichotomous variables as numbers and percentages. The T-test and Fisher's exact test were used to analyze differences in participant characteristics between those who developed MI during follow-up and those who did not. Logₑ-transformed miRNAs were used for all computations to approximate a Gaussian distribution. Correlations between miRNAs were assessed using Pearson correlation coefficients with Bonferroni-adjusted P-values. To further explore the complex inferences between plasma miRNAs, network analysis was applied as detailed above. Cox proportional hazard regression models were fitted to assess the association between logₑ-transformed miRNA levels and incident MI. To identify the subset or pattern of miRNA with the highest prognostic ability for future MI, two distinct approaches were used: (1) The first one was a two-step procedure. In order to reduce the number of candidate miRNAs and subsequent computational requirements forward and backward stepwise Cox regression analyses with relaxed in- and exclusion criteria (P_{entry}=0.15 and Pᵣₑₘₒᵥₐₗ=0.20; adjusted for age, sex and previous cardiovascular disease) were fitted. Eight MiRNAs selected in either or both of the analyses (miR-24, miR-126, miR-140, miR-150, miR-197, miR-223, miR-486, and miR-885-5p) were considered eligible for the second 'best subset' step. Cox regression models of all combinations of eligible miRNAs were computed and compared according to the models' Akaike information criterion (AIC) that is based on the maximized *log*-likelihood and imposes a penalty for increasing the number of parameters in the model. Lower values of AIC indicate the preferred model which is the one with the fewest parameters still providing adequate fit (tradeoff between accuracy and complexity). (2) The second approach utilized the technique called L₁-penalization implementing the 'least absolute shrinkage and selection operator [lasso] algorithm' to the 20 candidate miRNAs. L₁-penalized methods shrink the estimates of the regression coefficients towards zero relative to the maximum likelihood estimates. The technique has been employed to generate gene signatures from microarray data and prevents overfit arising from both collinearity and high-dimensionality. The amount of shrinkage is determined by the tuning parameter λ_{I}, which is progressively increased up to the value that shrinks all regression coefficients to zero. Plots of fitted regression coefficients (y-axis) versus λ_{I} (x-axis) were generated using the 'penalized' package of R statistical software (see Goeman J.J., L1 penalized estimation in the Cox proportional hazards model., Biom. J., 2010, 52(1):70-84). The lasso method allows assessing the relevance and robustness of individual explanatory variables but produces biased estimated for the regression coefficients. Accordingly, risk estimates for the three miRNAs finally selected (both approaches identified the same miRNA signature) were computed by standard Cox regression analysis and adjusted for age and sex (model 1), plus smoking status (ever vs. never smokers), systolic blood pressure, LDL cholesterol, diabetes and history of cardiovascular disease (model 2), plus other miRNAs (model 3), plus body mass index, waist-hip ratio, HDL cholesterol, logₑ C-reactive protein and fibrinogen (model 4). Two-sided P values below 0.05 were considered significant.

### RESULTS

### Plasma miRNAs in the Bruneck Study

Baseline demographic, clinical and laboratory characteristics of the 820 participants in the 1995 evaluation are shown in Table 1. All subjects were of Caucasian origin. A total of 47 participants experienced myocardial infarction over the 10-year follow-up period, corresponding to an incidence rate of 6.5 [95% CI 4.9-8.6] per 1000 person-years. For the initial screening, Human Taqman miRNA arrays (CardA v2.1 and CardB v2.0, Applied Biosystems) covering 754 small non-coding RNAs were applied to 8 pooled samples, consisting of subjects with and without atherosclerotic vascular disease matched for different cardiovascular risk factors (hypercholesterolemia, smoking, hypertension, diabetes). Of the 148 miRNAs with Ct-values < 36, 130 miRNAs were detected in human plasma using fluidic CardA and therefore all further analysis focused on this dataset. Eight plasma miRNAs that emerged as promising targets in the pre-screening and have previously been shown to display extreme spectra of node degrees, clustering coefficients, and eigenvector centrality values within the plasma miRNA network were selected and measured in the entire Bruneck cohort (n=820). Additional twelve miRNAs were quantified as part of an ongoing project on osteoarthritis (n=820). Finally, U6 was included as a normalization control.

As shown in Figure 6, miRNA levels were strongly correlated with each other, with some reaching almost perfect correlation (e.g. miR-24 and miR-223, r = 0.945). The complex dependency of miRNAs in participants who did and did not suffer MI was further scrutinized as miRNA-miRNA correlation profiles. Correlation patterns differed between both groups. A "re-wiring" of miRNA networks occurred around miR-126 and involved miR-197, miR-223, miR-24 and miR-885-5p.

### Association between plasma miRNAs and incident MI

MiRNA selection was based on two different approaches. (1) Stepwise Cox regression with comparison of AIC, a criterion considering both goodness of fit and the number of parameters in the model, identified three preferred combinations of miRNAs: miR-126/-197/-223, miR-126/-197/-24 and miR-126/-24/-885-5p (AIC ∼ 563 each with 6 degrees of freedom). (2) In L₁-penalized Cox regression analysis, miR-126, miR-197 and miR-223 showed the strongest association with incident MI at any level of penalization (λ₁) and emerged as the miRNAs requiring the highest λ₁ for their regression coefficients to be shrunk to zero whereas miR-24 had a worse performance.

Accordingly, the inventors gave preference to the miR-126/-197/-223 combination not containing miR-24. Risk estimated for the three finally selected miRNAs are given in Figure 7. MiR-223 and miR-197 showed positive associations with MI (multivariable hazard ratios 2.19 [95% CI 1.26-3.83], P=0.006, and 1.79 [95% CI 1.00-3.20], P=0.049) while miR-126 was inversely related to disease risk (multivariable hazard ratio 0.40 [95% CI 0.22-0.73], P=0.003). There was no effect modification by sex, diabetes or pre-existing cardiovascular disease (Table 2). When the other miRNAs were individually added to the multivariable model already including miR-126/-197/-223, none achieved statistical significance.

### The cellular origin of plasma miRNAs

To determine the cellular origin of the three plasma miRNAs of interest, expression levels were compared in PBMCs, platelets and HUVECs. As expected, miR-126 was highly enriched in endothelial cells (Figure 8A). In contrast, miR-223 was abundant in PBMCs and platelets (Figure 8B), whereas miR-197 was similarly expressed in all three cell types (Figure 8C).

### Proteomic analysis to identify miR-126 targets

To screen for potential direct and indirect effects of miR-126 on the proteome of endothelial cells, the inventors employed difference in-gel electrophoresis (DIGE) which is capable of detecting differences in protein expression as low as 10%. HUVECs were transfected with the precursor of miR-126 (premiR126) or pre-miRNA controls. The overexpression of miR-126 was confirmed by qPCR. After 48h, the cells were harvested and the proteome was compared by DIGE. Differentially expressed protein spots were excised (Figure 9A), digested with trypsin and identified by LC-MS/MS. The proteomic approach was combined with open access bioinformatics methodology (open source predictive software programs miRBase, TargetScan, miRNAviewer, MIRanda, PicTar) to discern direct and indirect effects of miR-126. Only procollagen-lysine, 2-oxoglutarate 5-dioxygenase 2 (PLOD2 HUMAN) had an almost perfect Watson-Crick base pairing in the seed region (one G:U wobble).

### MiR-126 and secretion of PAI-1

In the context of MI, the differential expression of plasminogen activator inhibitor 1 (PAI1_HUMAN) was of particular interest. PAI-1 is a potent inhibitor of fibrinolysis. Consistent with previous reports, regulation of PAI-1 by miR-126 did not occur through mRNA degradation or translational inhibition (Figure 9B and 9C). DIGE, however, also visualizes changes in post-translational modifications. Thus, regulation of PAI-1 by miR-126 may result from post-translational modifications, which could alter endothelial PAI-1 secretion. Indeed, HUVECs transfected with premiR-126 secreted significantly less PAI-1 (Figure 9D) accounting for reduced PAI activity in their conditioned medium (Figure 9E).

### DISCUSSION

Expression signatures of miRNAs are emerging as an exciting tool for assessing risk associated with distinct pathological conditions. Plasma miRNAs packaged in membranous microvesicles mainly derived from leukocytes (∼40%), platelets (∼30%) and endothelial cells (∼10%). This is the first prospective, population-based study on the association between plasma miRNAs and incident MI. The present data highlights a "rewiring" of plasma miRNAs around endothelial miR-126 in subjects with subsequent MI. Moreover, the inventors provide evidence that miR-126 expression enhances the fibrinolytic properties of the endothelium providing a potential explanation for its inverse relation with MI risk.

### Plasma miRNAs in myocardial infarction

Previously, miRNAs enriched in myocytes, such as miR-1, miR-133a, miR-133b, miR-499-5p and the cardiac specific miR-208a were reported to be released from damaged muscle and detectable after acute MI.¹² In healthy individuals, these miRNAs are undetectable in plasma or present in very low copy numbers.¹² Thus, they might serve as potential biomarkers of acute cardiac damage, but cannot be used for risk assessment in healthy individuals. The inventors aim was to identify changes in circulating miRNAs that might precede future cardiac events. For this purpose, the inventors utilized plasma samples from the 1995 evaluation of the Bruneck cohort and analyzed the potential association between baseline miRNA levels and incidence of MI over a 10-year observation period (1995 to 2005, median time until MI = 72 months). Because most plasma miRNAs were highly correlated, global patterns of expression should be studied by representing miRNA data as co-expression networks. In the analysis, the inventors considered 8 miRNAs that emerged as promising targets in the pre-screening and displayed unique network topology. Three of these miRNAs formed part of a miRNA signature for MI: miR-126, miR-197 and miR-223. Findings were independent of classic vascular risk factors, stable in subgroups (men and women, diabetics and non-diabetics, participants with and without previous cardiovascular disease) (Table 2) and robust when using distinct statistical approaches. Another 12 miRNAs were not related to atherosclerotic vascular disease in the pre-screening and fell short of significance in the main analysis.

### MiR-126 and fibrinolysis

Although miR-126 is highly enriched in endothelial cells, it is also detectable in other cell types such as platelets and PBMCs, albeit at lower concentrations (Figure 8). Hence, adjustment for other miRNAs can refine the endothelial contribution to the miR-126 content in plasma. MiR-126 and its expression have previously been linked to vascular integrity and endothelial cell homeostasis.²²⁻²⁴ Confirmed targets of miR-126 are the Sprouty-related EVHI domain-containing protein-1 (SPREDI), which is an intracellular inhibitor of angiogenic signalling, the phosphoinositol-3 kinase regulatory subunit 2 (PIK3R2) and the vascular cell adhesion molecule 1 (VCAM-1). Here the inventors reveal a novel role of miR-126 in the regulation of fibrinolysis. Using proteomics, the inventors discovered that overexpression of miR-126 in endothelial cells significantly reduced the secretion of PAI-1, the primary inhibitor of endogenous fibrinolysis afforded by urokinase-type and tissue-type plasminogen activators (uPA and tPA).⁴¹ Low levels of miR-126 may thus translate into high PAI-1 activity and impaired fibrinolysis. Actually, high PAI-1 plasma activity has been linked to an elevated risk of athero-thrombotic cardiovascular events,⁴¹⁻⁴⁵ may determine resistance to standard recanalyzing therapy (thrombolysis) and constitute a key pathophysiological component in symptomatic diabetic macroangiopathy. Failure of standard anti-diabetic therapies like sulfonylureas and insulin to normalize PAI-1 levels has been suggested to partly explain their disappointing effects on vascular risk.

### MiR-197 and MiR-223

MiR-223 is more abundant in platelets and PBMCs than endothelial cells, whereas miR-197 is expressed at similar levels in all three cell types (Figure 8). Currently, knowledge about potential vascular effects of both miRNAs is limited. MiR-223 has been shown to negatively regulate progenitor cell function and granulocyte function and miR-24, which is highly correlated with miR-223, is a p53-independent cell cycle inhibitor.

### Conclusions

This study demonstrates that plasma miRNAs are associated with the risk of MI in the general population and that miR-126 might be pathophysiotogically linked to secretion of PAI-1, the key inhibitor of endogenous fibrinolysis.

### Example 3

Additional analysis from the Bruneck study has now been performed. In particular, data from the 2010 evaluations has been included. So the participants were followed-up for 15 (1995-2010) rather than 10 years (1995-2005) and there are an additional 20% more cases.

Statistical analysis of the results was performed as described above for Example 2 (data not shown).

The association for miR-126 and miR-223 still holds even over a 15 year observation period and is more pronounced in patients with fatal than non-fatal myocardial infarction. In particular, the combination of miR-126 and miR-223 provides an effective method for predicting and diagnosing cardiovascular disorder. For miR-197, there was a reduction in significance suggesting that among the 3 miRNAs this is the weakest marker.

**Table 1. Baseline characteristics of the study population.**

| **Variables** | **All participants** | **Incident myocardial infarction** | | **P value** |
|---|---|---|---|---|
| | (n=820) | Yes (n=47) | No (n=773) | |
| **Demographics and life-style** | | | | |
| Age, years | 62.9 (11.1) | 70.0 (9.6) | 62.4 (11.1) | <0.001 |
| Female sex, n (%) | 409 (49.9) | 17 (36.2) | 392 (50.7) | 0.070 |
| Current/ex-smoker, n (%) | 372 (45.4) | 23 (48.9) | 349 (45.2) | 0.652 |
| Alcohol consumption, g/d | 23.8 (31.2) | 25.0 (34.1) | 23.7 (31.0) | 0.781 |

| **Physical examination** | | | | |
|---|---|---|---|---|
| Body mass index, kg/m² | 25.7 (3.9) | 26.4 (4.9) | 25.6 (3.8) | 0.165 |
| Waist-hip ratio, cm/cm | 0.9 (0.1) | 1.0 (0.1) | 0.9 (0.1) | 0.012 |
| Systolic blood pressure, mmHg | 148.2 (20.6) | 155.9 (22.9) | 147.7 (20.4) | 0.008 |
| Diastolic blood pressure, | 87.1 (9.1) | 87.7 (8.9) | 87.0 (9.2) | 0.646 |
| mmHg | | | | |

| **Lipid markers** | | | | |
|---|---|---|---|---|
| Total cholesterol, mmol/L | 6.0 (1.1) | 6.4 (1.4) | 5.9 (1.1) | 0.003 |
| HDL cholesterol, mmol/L | 1.5 (0.4) | 1.5 (0.5) | 1.5 (0.4) | 0.762 |
| LDL cholesterol, mmol/L | 3.8 (1.0) | 4.1 (1.3) | 3.7 (1.0) | 0.033 |
| Triglycerides, mmol/L* | 1.3 (1.7) | 1.4 (1.7) | 1.3 (1.7) | 0.223 |
| Apolipoprotein A-1, g/L | 1.7 (0.3) | 1.7 (0.3) | 1.7 (0.3) | 0.926 |
| Apolipoprotein B, g/L | 1.2 (0.3) | 1.3 (0.4) | 1.2 (0.3) | 0.004 |

| **Inflammatory markers** | | | | |
|---|---|---|---|---|
| Leukocytes, x 10⁹/L | 6.5 (1.7) | 6.9 (1.5) | 6.5 (1.7) | 0.111 |
| C-reactive protein, nmol/L* | 17.1 (2.8) | 22.2(2.5) | 16.8 (2.8) | 0.070 |
| Fibrinogen, g/L | 2.9 (0.8) | 3.1 (0.6) | 2.9 (0.8) | 0.023 |

| **Diabetes** | | | | |
|---|---|---|---|---|
| Type 2 Diabetes, n (%) | 82 (10.0) | 6 (12.8) | 76(9.8) | 0.457 |
| HbAlc, % | 5.8 (3.7) | 6.7 (7.5) | 5.7 (3.4) | 0.074 |

| **Previous diseases** | | | | |
|---|---|---|---|---|
| Cardiovascular diseases, n (%) | 57 (7.0) | 12 (25.5) | 45 (5.8) | <0.001 |

| | | | | |
|---|---|---|---|---|
| Values are means (SD) or numbers (percentages) unless indicated otherwise. * Variables were logₑ-transformed for analysis and are presented as geometric mean (geometric SD). | | | | |

**Table 2. Interaction analysis of the association between selective miRNAs and myocardial infarction (MI).**

| **Subgroups** | **Hazard ratio (95% CI) for myocardial infarction per 1 SD Increase of logₑ miRNA*** | | |
|---|---|---|---|
| | **MiR-126** | **MiR-197** | **MiR-223** |
| **Diagnosis of diabetes** | | | |
| Yes | 0.23 (0.09-0.61) | 1.06 (0.36-3.14) | 1.46 (0.61-3.51) |
| No | 0.43 (0.23-0.79) | 1.86 (1.04-3.34) | 2.31 (1.31-4.07) |
| | P_{interaction}=0.131 | P_{interaction}=0.268 | P_{interaction}=0.286 |

| **Sex** | | | |
|---|---|---|---|
| Male | 0.39 (0.21-0.73) | 1.57 (0.86-2.89) | 1.95 (1.05-3.63) |
| Female | 0.42 (0.20-0.90) | 2.50 (1.14-5.51) | 2.68 (1.30-5.53) |
| | P_{interaction} =0.814 | P_{interaction}=0.209 | P_{interaction}=0.392 |

| **History of cardiovascular disease** | | | |
|---|---|---|---|
| Yes | 0.41 (0.22-0.76) | 2.02 (1.10-3.70) | 2.29 (1.28-4.08) |
| No | 0.30 (0.12-0.79) | 1.19 (0.53-2.70) | 1.83 (0.74-4.54) |
| | P_{interaction}=0.486 | P_{interaction}=0.189 | P_{interaction}=0.624 |

| | | | |
|---|---|---|---|
| * All Cox models included an interaction term of diabetes, sex or history of cardiovascular disease with the miRNA of interest, plus the variables age, sex, smoking status (ever vs. never smokers), systolic blood pressure, LDL cholesterol, diabetes, history of cardiovascular disease, miR-126, miR-197 and miR-223. | | | |

**Table 3: Protein changes in HUVECs after overexpressing pre-miR126**

| **Spot No.** | **Accession No.** | **Entry Name** | **Protein Name** | **Mw (kDa)** | **Coverage (%)** | **Unique Peptides** | **Unique Spectra** | **Assigned Spectra** | **Ratio** | **P value** |
|---|---|---|---|---|---|---|---|---|---|---|
| 1 | P21333 | FLNA_HUMAN | Filamin-A | 280,711 | 11.10% | 21 | 27 | 37 | -1.12 | 0.0003 |
| 2 | P21333 | FLNA_HUMAN | Filamin-A | 280,711 | 8.73% | 16 | 20 | 30 | -1.15 | 0.015 |
| 3 | P21333 | FLNA_HUMAN | Filamin-A | 280,711 | 10.50% | 20 | 25 | 34 | -1.18 | 0.0018 |
| 4 | 075369 | FLNB_HUMAN | Filamin-B | 278,172 | 8.07% | 14 | 17 | 23 | -1.17 | 0.00057 |
| 5 | 075369 | PLMB_HUMAN | Filamin-B | 278,172 | 7.11% | 13 | 14 | 20 | -1.20 | 0.038 |
| 5 | Q8WUM4 | PDC61_HUMAN | Programmed cell death 6-interacting protein | 96,007 | 14,30% | 11 | 11 | 17 | -1.20 | 0.038 |
| 6 | Q09666 | AHNK_HUMAN | Neuroblast differentiation-associated protein AHNAK | 629,086 | 3.24% | 14 | 15 | 21 | -1.23 | 0.0021 |
| 6 | O75369 | FLNB_HUMAN | Filamin-B | 278,172 | 6.11% | 11 | 13 | 21 | -1.23 | 0.0021 |
| 6 | Q8WUM4 | PDC61_HUMAN | Pogrammed cell death 6-interacting protein | 96,007 | 19.70% | 13 | 15 | 24 | -1.23 | 0.0021 |
| 7 | Q8WUM4 | PDC61_HUMAN | Programmed cell death 6-interacting protein | 96,007 | 11.60% | 9 | 9 | 14 | -1.24 | 0.012 |
| 7 | 000469 | PLOD2_HUMAN | Procollagen-lysine,2-oxoglutarate 5-dioxygenase 2 | 84,669 | 11.10% | 6 | 8 | 13 | -1,24 | 0.012 |
| 7 | P02787 | TRFE_HUMAN | Serotransferrin | 77,032 | 6.59% | 4 | 4 | 7 | -1.24 | 0.012 |
| 8 | Q09666 | AHNK_HUMAN | Neuroblast differential-associated protein AHNAK | 629,086 | 1.02% | 5 | 5 | 6 | -1.21 | 0.0084 |
| 8 | Q8WUM4 | PDC61_HUMAN | Programmed cell death 6-interacting protein | 96,007 | 4.95% | 4 | 4 | 7 | -1.21 | 0.0084 |
| 8 | 000469 | PLOD2_HUMAN | Procollagen-lysine,2-oxoglutarate 5-dioxygenase 2 | 84,669 | 5.70% | 3 | 5 | 7 | -1.21 | 0.0084 |
| 9 | P08107 | HSP71_HUMAN | Heat shock 70 kDa protein 1 | 70,036 | 27.30% | 15 | 18 | 31 | -1.26 | 0.032 |
| 10 | P02545 | LMMA_HUMAN | Lamin-A/C | 74,123 | 30.00% | 21 | 24 | 33 | -1.26 | 0.037 |
| 11 | P02545 | LMNA_HUMAN | Lamin-A/C | 74,123 | 37.70% | 24 | 26 | 39 | -1.20 | 0.042 |
| 12 | P02545 | LMNA_HUMAN | Lamin-A/C | 74,123 | 33.00% | 21 | 22 | 34 | -1.14 | 0.035 |
| 13 | 060506 | HNRPQ_HUMAN | Heterogeneous nuclear ribonucleoprotein Q | 69,586 | 8.51% | 7 | 7 | 13 | 1.22 | 0.045 |
| 14 | 060506 | HNRPQ_HUMAN | Heterogeneous nuclear ribonucleoprotein Q | 69,586 | 15.60% | 10 | 11 | 19 | -1.12 | 0.015 |
| 15 | P14625 | ENPL_HUMAN | Endoplasmin | 92,454 | 17.40% | 11 | 13 | 28 | -1.15 | 0.019 |
| 16 | P14625 | ENPL_HUMAN | Endoplasmin | 92,454 | 13.000Æ. | 8 | 9 | 21 | 1.17 | 0.044 |
| 17 | P14625 | ENPL_HUMAN | Endoplasmin | 92,454 | 11.00% | 7 | 8 | 16 | 1.13 | 0.04 |
| 18 | P31943 | HNRHI_HUMAN | Heterogeneous nuclear ribonucleoprotein H | 49,212 | 20.70% | 7 | 8 | 11 | -1.18 | 0039 |
| 18 | P35998 | PRS7_HUMAM | 26S protease regulatory subnit 7 | 48,618 | 16.40% | 6 | 6 | 10 | -1.1819 | 0.039 |
| 19 | P55809 | SCOTI_HUMAN | Succinyl-CoA:3-ketoacid-coenzyme A transferase 1, mitochondrial | 56,141 | 12.10% | 5 | 6 | 8 | -1.14 | 0.015 |
| 19 | P78371 | TCPB_HUMAN | T-complex protein 1 subunit beta | 57,472 | 10.50% | 4 | 4 | 8 | -1.14 | 0.015 |
| 20 | Q9NVA2 | SEP11_HUMAN | Septin-11 | 49,381 | 21.90% | 8 | 9 | 12 | 1.18 | 0.016 |
| 21 | P49411 | EFTU_HUMAN | Elongation factor Tu, mitochondrial | 49,524 | 22.30% | 8 | 9 | 13 | 1.18 | 0.049 |
| 21 | P05121 | PA11_HUMAN | Plasminogen activator inhibitor 1 | 45,042 | 14.40% | 5 | 6 | 10 | 1.18 | 0.049 |
| 22 | P49411 | EFTU_HUMAN | Elongation factor Tu, mutochondrial | 49,524 | 30.10% | 11 | 11 | 20 | 1.3 | 0.036 |
| 23 | P05121 | PA11_HUMAN | Plasminogen activator inhibitor 1 | 45,042 | 27.90% | 10 | 12 | 21 | 1.22 | 0.00023 |
| 23 | P62195 | PRS8_HUMAN | 26S protease regulatory subnit 8 | 45,609 | 24.90% | 8 | 9 | 18 | 1.22 | 0.00023 |
| 24 | Q15417 | CNN3_HUMAN | Calponin-3 | 36,397 | 11.60% | 4 | 4 | 5 | 1.17 | 0.041 |
| 25 | P12004 | PCNA_HUMAN | Proliferating cell nuclear antigen | 28,751 | 31.80% | 6 | 6 | 13 | 1.17 | 0.012 |
| 25 | Q6NZ12 | PTRF_HUMAN | Polymerase 1 and transcript release factor | 43,459 | 15.10% | 5 | 6 | 14 | 1.17 | 0.012 |
| 25 | P08670 | VIME_HUMAN | Vimentin | 53,635 | 18.70% | 7 | 8 | 13 | 1.17 | 0.012 |
| 26 | Q07065 | CKAP4_HUMAN | Cytoskeleton-associated protein 4 | 66,004 | 10.10% | 4 | 4 | 5 | 1.13 | 0.034 |
| 26 | P29692 | EFID_HUMAN | Elongation factor 1-delta | 31,104 | 22.80% | 5 | 5 | 6 | 1.13 | 0.034 |
| 27 | P11021 | GRP78_HUMAN | 78 kDa glucose-regulated protein | 72,317 | 16.70% | 8 | 9 | 13 | 1.20 | 0.0024 |
| 28 | P07355 | ANXA2_HUMAN | Annexin A2 | 38,588 | 58.10% | 24 | 30 | 62 | -1.23 | 0.028 |
| 29 | P40926 | MDHM_HUMAN | Malate dehydrogenase, mitochondrial | 35,486 | 21.90% | 6 | 6 | 10 | 1.25 | 0.011 |
| 30 | P63244 | GBLP_HUMAN | Guanine nucleotide-binding protein subunit beta-2-like 1 | 35,059 | 38.50% | 9 | 9 | 16 | -1.18 | 0.0084 |
| 30 | P32322 | P5CR1_HUMAN | Pyrroline-5-carboxylate reductase 1, mitochondrial | 33,343 | 33.20% | 9 | 10 | 16 | -1.18 | 0.0084 |
| 30 | P45880 | VDAC2_HUMAN | Voltage-dependent anion-selective channel protein | 31,549 | 26.50% | 7 | 8 | 15 | -1.18 | 0.0084 |
| 31 | P11021 | GRP78_HUMAN | 78 kDa glucose-regulated protein | 72,317 | 11.50% | 5 | 6 | 8 | 1.30 | 0.031 |
| 31 | P21796 | VDAC1_HUMAN | Voltage-dependent anion-selective channel protein 1 | 30,756 | 15.50% | 4 | 4 | 6 | 1.30 | 0.031 |
| 32 | P21796 | VDAC1_HUMAN | Voltage-dependent anion-selective channel protein 1 | 30,756 | 19.40% | 5 | 6 | 10 | 1.35 | 0.03 |
| 33 | Q9H7Z7 | PGES2_HUMAN | Prostaglandin E synthase 2 | 41,926 | 5.57% | 2 | 2 | 5 | 1.16 | 0.032 |
| 34 | P21796 | VDAC1_HUMAN | Voltage-dependent anion-selective channel protein 1 | 30,756 | 38.90% | 8 | 10 | 13 | -1.19 | 0.025 |
| 35 | P28066 | PSA5-HUMAN | Proteasome subunit alpha type-5 | 26,393 | 37.30% | 7 | 10 | 19 | 1.11 | 0.045 |
| 36 | P07858 | CATB_HUMAN | Cathepsin B | 37,803 | 8.26% | 2 | 2 | 3 | 1.25 | 0.023 |
| 36 | Q07065 | CKAP4_HUMAN | Cytoskeleton-associated protein 4 | 66,004 | 4.82% | 2 | 2 | 3 | 1.25 | 0.023 |
| 36 | P08670 | VIME_HUMAN | Vimentin | 53,635 | 8.3 7% | 3 | 3 | 3 | 1.25 | 0.023 |
| 37 | P08670 | VIME_HUMAN | Vimentin | 53,635 | 17.60% | 9 | 9 | 14 | 1.19 | 0.00082 |
| 38 | P60709 | ACTB_HUMAN | Actin, cytoplasmic 1 | 41,776 | 15.50% | 5 | 5 | 9 | 1.20 | 0.027 |
| 38 | P08133 | ANXA6_HUMAM | Annexin A6 | 75,860 | 9.66% | 6 | 7 | 9 | 1.20 | 0.027 |
| 38 | P08670 | VIME_HUMAN | Vimentin | 53,635 | 12.40% | 5 | 5 | 8 | 1.20 | 0.027 |
| 39 | P06576 | ATPB_HUMAN | ATP synthase subunit beta, mitochondrial | 56,543 | 14.90% | 6 | 6 | 12 | 1.18 | 0.024 |
| 39 | Q13162 | PRDX4_HUMAM | Peroxiredoxin-4 | 30,523 | 30.60% | 7 | 8 | 10 | 1.18 | 0.024 |
| 40 | 075489 | NDUS3_HUMAN | NADH dehydrogenase [ubiquinone] iron-sulfur protein 3, mitochondrial | 30,224 | 20.10% | 7 | 11 | 19 | 1.17 | 0.025 |
| 41 | P30084 | ECHM_HUMAN | Enoyl-CoA hydratase, mitochondrial | 31,370 | 23,40% | 6 | 7 | 12 | 1.16 | 0.031 |
| 41 | P35232 | PHB_HUMAN | Prohibition | 29,787 | 20.20% | 5 | 6 | 10 | 1.16 | 0.031 |
| 42 | P30040 | ERP29_HUMAN | Endoplasmic reticulum protein ERp29 | 28,977 | 26.40% | 6 | 6 | 8 | 1.11 | 0.023 |
| 42 | Q99436 | PSB7_HUMAN | Proteasome subunit beta type-7 | 29,948 | 8.30% | 3 | 4 | 7 | 1.11 | 0.023 |
| 43 | P06576 | ATPB_HUMAN | ATP synthase subunit beta, mitochondrial | 56,543 | 9.83% | 4 | 4 | 6 | 1.19 | 0.027 |
| 43 | 095571 | ETHE1_HUMAN | Protein ETHE1, mitochondrial | 27,855 | 20.90% | 4 | 4 | 6 | 1.19 | 0.027 |
| 44 | Q9Y224 | CN166_HUMAM | UPF0568 protein C14orf166 | 28,051 | 10.70% | 3 | 3 | 6 | 1.29 | 0.0024 |
| 44 | 095571 | ETHE1_HUMAN | Protein ETHE1, mitochondrial | 27,855 | 20.90% | 4 | 4 | 5 | 1.29 | 0.0024 |
| 45 | P07355 | ANXA2_HUMAN | Annexin A2 | 38,588 | 5.90% | 2 | 2 | 3 | 1.27 | 0.01 |
| 46 | P30048 | PRDX3_HUMAN | Thioredoxin-dependent peroxide reductase, mitochondrial | 27,675 | 25,40% | 6 | 6 | 10 | 1.20 | 0.045 |
| 47 | 043809 | CPSF5_HUMAN | Cleavage and polyadenylation specificity factor subunit 5 | 26,210 | 16.70% | 4 | 4 | 7 | 1.21 | 0.033 |
| 47 | P08670 | VIME_HUMAN | Vimentin | 53,635 | 13.50% | 7 | 8 | 13 | 1.21 | 0.033 |
| 48 | P08670 | VIME_HUMAN | Vimentin | 53,635 | 12.40% | 5 | 6 | 10 | 1.32 | 0.05 |
| 49 | P01024 | CO3_HUMAN | Complement C3 | 187,131 | 1.44% | 2 | 2 | 4 | 1.18 | 0.024 |
| 50 | | | Unidentified | - | | | | | -1.23 | 0.025 |

### References

1. Bartel DP. Cell. 2009 Jan 23; 136(2):215-33.
2. Pillai RS, Bhattacharyya SN, Filipowicz W. Trends Cell Biol. 2007 Mar;17(3):118-26.
3. Kloosterman WP, Plasterk RH. Dev Cell. 2006 Oct;11(4):441-50.
4. Stefani G, Slack FJ. Nat Rev Mol Cell Biol. 2008 Mar;9(3):219-30.
5. Latronico MV, Catalucci D, Condorelli G. Circ Res. 2007 Dec 7;101(12):1225-36.
6. van Rooij E, Marshall WS, Olson EN. Circ Res. 2008 Oct 24;103(9):919-28.
7. Kuehbacher A, Urbich C, Zeiher AM, Dimmeler S. Circ Res. 2007 Jul 6;101(1):59-68.
8. Mitchell PS et al. Proc Natl Acad Sci USA. 2008 Jul 29;105(30):10513-8.
9. Tanaka M et al. PLoS One. 2009;4(5):e5532.
10. Laterza OF et al. Clin Chem. 2009 Nov;55(11):1977-83.
11. Wang K et al. Proc Natl Acad Sci USA. 2009 Mar 17;106(11):4402-7.
12. Wang GK et al. Eur Heart J. 2010 Mar;31(6):659-66.
13. Ji X, Takahashi R, Hiura Y, Hirokawa G, Fukushima Y, Iwai N. Clin Chem. 2009 Nov;55(11):1944-9.
14. Tijsen AJ et al. Circ Res. 2010 Apr 2;106(6):1035-9.
15. Nathan DM. Long-term complications of diabetes mellitus. N Engl J Med. 1993 Jun 10;328(23):1676-85.
16. Frankel DS et al. Circulation. 2008 Dec 9;118(24):2533-9.
17. Kiechl S et al. N Engl J Med. 2002 Jul 18;347(3):185-92.
18. Bonora E et al. Diabetes. 2004 Jul;53(7):1782-9.
19. Kiechl S et al. Circulation. 2007 Jul 24;116(4):385-91.
20. Faith JJ et al. PLoS Biol. 2007 Jan;5(1);e8.
21. van Dongen S. Graph clustering by flow simulation 2000.
22. Chuang HY, Lee E, Liu YT, Lee D, Ideker T. Mol Syst Biol. 2007;3:140.
23. Fish JE et al. Dev Cell. 2008 Aug;15(2):272-84.
24. Wang S et al. Dev Cell. 2008 Aug;15(2):261-71.
25. VanWijk MJ, VanBavel E, Sturk A, Nieuwland R. Cardiovasc Res. 2003 Aug 1;59(2):277-87.
26. Volinia S et al. Genome Res. 2010 May;20(5):589-99.
27. Jeong H et al. Nature. 2001 May 3;411(6833):41-2.
28. Zernecke A et al. Sci Signal. 2009 Dec 8;2(100):ra81.
29. Prokopi M et al. Blood. 2009 Jul 16;114(3):723-32.
30. Waltenberger J, Lange J, Kranz A. Circulation. 2000 Jul 11;102(2):185-90.
31. Kiechl S et al. Arterioscler Thromb Vasc Biol. 2007 Aug;27(8):1788-95.
32. Tsimikas S et al. Eur Heart J. 2009 Jan;30(1).107-15.
33. Baecke JA et al. Am J Clin Nutr. 1982 Nov;36(5):936-42.
34. Ramakers C et al. Neurosci Lett. 2003 Mar 13;339(1):62-6.
35. Hosmer DW LS. Applied Logistic Regression; 2000.
36. Roulston M. Physica D. 1997(110):62.6.
37. Slonim N et al. Proc Natl Acad Sci USA. 2005 Dec 20;102(51):18297-302.
38. Bansal et al. Mol Syst Biol. 2007;3:78.
39. Barabasi AL. Science. 2009 Jul 24;325(5939):412-3.
40. Enright AJ et al. Nucleic Acids Res. 2002 Apr 1;30(7):1575-84.
41. Diebold I et al. Thromb Haemost. 2008 Dec;100(6):984-91.
42. Smith A et al. The Caerphilly Study. Circulation. 2005 Nov 15;112(20):3080-7.
43. Kohler HP et al. N. Engl. J. Med. 2000 Jun 15;342(24):1792-801.
44. Thogersen AM et al. Circulation. 1998 Nov 24;98(21):2241-7.
45. Hamsten A et al. Lancet. 1987 Jul 4;2(8549):3-9.

The present disclosure relates also to the following clauses :
1. A method of predicting and/or diagnosing a cardiovascular disorder comprising determining in a sample obtained from an individual the level of at least 2 microRNAs selected from the group consisting of miR-126, miR-223 and miR-197 or selected from the group consisting of miR-126, miR-24 and miR-197.
2. The method according to clause 1, wherein the level of:
   i. miR-126 and miR-223; or
   ii. miR-126 and miR-24,
   are determined.
3. The method according to clause 1, wherein the level of miR-126 and miR-223 are determined.
4. The method of clause 2 or clause 3, wherein the level of miR-197 is additionally determined.
5. A method of determining whether an individual has diabetes comprising determining in a sample obtained from the individual the level of the following microRNAs: miR-15a, miR-126, miR223, miR-320 and miR-28-3p.
6. A method of predicting diabetes comprising determining in a sample obtained from an individual the level of the following microRNAs: miR-15a, miR-29b, miR-126, miR223 and miR-28-3p.
7. The method according to clause 5 additionally comprising determining the level of one or more of the following mircoRNAs: miR-20b, miR-21, miR-24, miR-29b, miR-191, miR-197, miR-486 and miR-150.
8. The method according to any one of the preceding clauses, wherein the sample is a tissue sample, blood or plasma.
9. The method of clause 8, wherein the sample is plasma.
10. The method according to clause 5, wherein an individual is determined to have diabetes by a low level of miR-15a, miR-126, miR223 and miR-320, and a high level of miR-28-3p.
11. The method according to clause 7, wherein an individual is determined to have diabetes by a low level of miR-15a, miR-126, miR223 and miR-320, and a high level of miR-28-3p, and a low level of one or more of miR-20b, miR-21, miR-24, miR-29b, miR-191, miR-197, miR-486 and miR-150.
12. The method according to clause 6, wherein a positive prediction of diabetes is given by a low level of miR-15a, miR-29b, miR-126 and miR223, and a high level of miR-28-3p.
13. The method according to any one of clauses 5 and 6, wherein the diabetes is type II diabetes.
14. A sensor for detecting the levels of at least 2 of the following microRNAs:
   i. miR-126, miR-223 and miR-197; or
   ii. miR-126, miR-24 and miR-197.
15. A sensor for detecting the levels of the following microRNAs: miR-15a, miR-126, miR223, miR-320 and miR-28-3p.
16. A sensor for detecting the levels of the following microRNAs: miR-15a, miR-29b, miR-126, miR223 and miR-28-3p.
17. The sensor of clause 15, which is additionally for detecting the levels of one or more of the following mircoRNAs: miR-20b, miR-21, miR-24, miR-29b, miR-191, miR-197, miR-486 and miR-150.
18. A kit comprising reagents for detecting the level of at least 2 of the following microRNAs:
   i. miR-126, miR-223 and miR-197; or
   ii. miR-126, miR-24 and miR-197.
19. A kit comprising reagents for detecting the level of the following microRNAs: miR-15a, miR-126, miR223, miR-320 and miR-28-3p.
20. A kit comprising reagents for detecting the level of the following microRNAs: miR-15a, miR-29b, miR-126, miR223 and miR-28-3p.
21. The kit of clause 19 further comprising reagents for detecting the level of one or more of the following mircoRNAs: miR-20b, miR-21, miR-24, miR-29b, miR-191, miR-197, miR-486 and miR-150.
22. The kit of any one of clauses 18 to 21, which comprises reagents for detecting the level of the microRNAs by RT-PCR.
23. A method of predicting or diagnosing a vasculature disorder comprising determining in a sample obtained from an individual the level of microRNA MiR-126.

## Claims

1. A method of predicting myocardial infarction comprising determining in a sample obtained from an individual the level of at least 2 microRNAs selected from the group consisting of miR-126, miR-223 and miR-197 or selected from the group consisting of miR-126, miR-24 and miR-197.

2. The method according to claim 1, wherein the level of:
i. miR-126 and miR-223; or
ii. miR-126 and miR-24,
are determined.

3. The method according to claim 1, wherein the level of miR-126 and miR-223 are determined.

4. The method of claim 2 or claim 3, wherein the level of miR-197 is additionally determined.

5. A method of determining whether an individual has type II diabetes comprising determining in a sample obtained from the individual the level of the following microRNAs: miR-15a, miR-126, miR223, miR-320 and miR-28-3p.

6. A method of predicting type II diabetes comprising determining in a sample obtained from an individual the level of the following microRNAs: miR-15a, miR-29b, miR-126, miR223 and miR-28-3p.

7. The method according to claim 5 additionally comprising determining the level of one or more of the following mircoRNAs: miR-20b, miR-21, miR-24, miR-29b, miR-191, miR-197, miR-486 and miR-150.

8. The method according to any one of the preceding claims, wherein the sample is a tissue sample, blood or plasma.

9. The method of claim 8, wherein the sample is plasma.

10. The method according to claim 5, wherein an individual is determined to have type II diabetes by a low level of miR-15a, miR-126, miR223 and miR-320, and a high level of miR-28-3p.

11. The method according to claim 7, wherein an individual is determined to have type II diabetes by a low level of miR-15a, miR-126, miR223 and miR-320, and a high level of miR-28-3p, and a low level of one or more of miR-20b, miR-21, miR-24, miR-29b, miR-191, miR-197, miR-486 and miR-150.

12. The method according to claim 6, wherein a positive prediction of type II diabetes is given by a low level of miR-15a, miR-29b, miR-126 and miR223, and a high level of miR-28-3p.

## Patentansprüche

1. Verfahren zum Vorhersagen eines Herzinfarkts umfassend Bestimmen des Levels von mindestens 2 microRNAs, die aus der Gruppe bestehend aus miR-126, miR-223 und miR-197 ausgewählt sind, oder die aus der Gruppe bestehend aus miR-126, miR-24 und miR-197 ausgewählt sind, in einer Probe, die aus einem Individuum gewonnen wurde.

2. Verfahren gemäß Anspruch 1, wobei die Level von:
i. miR-126 und miR-223; oder
ii. miR-126 und miR-24,
bestimmt werden.

3. Verfahren gemäß Anspruch 1, wobei die Level von miR-126 und miR-223 bestimmt werden.

4. Verfahren nach Anspruch 2 oder Anspruch 3, wobei zusätzlich das Level von miR-197 bestimmt wird.

5. Verfahren zum Bestimmen, ob ein Individuum Typ-2-Diabetes aufweist, umfassend Bestimmen des Levels der folgenden microRNAs: miR-15a, miR-126, miR223, miR-320 und miR-28-3p in einer Probe, die aus einem Individuum gewonnen wurde.

6. Verfahren zum Vorhersagen von Typ-2-Diabetes umfassend Bestimmen des Levels der folgenden microRNAs: miR-15a, miR-29b, miR-126, miR223 und miR-28-3p in einer Probe, die aus einem Individuum gewonnen wurde.

7. Verfahren gemäß Anspruch 5, zusätzlich umfassend Bestimmen des Levels von einer oder mehreren der folgenden microRNAs: miR-20b, miR-21, miR-24, miR-29b, miR-191, miR-197, miR-486 und miR-150.

8. Verfahren gemäß einem der vorhergehenden Ansprüche, wobei die Probe eine Gewebeprobe, Blut oder Plasma ist.

9. Verfahren nach Anspruch 8, wobei die Probe Plasma ist.

10. Verfahren gemäß Anspruch 5, wobei ein Individuum durch ein niedriges Level von miR-15a, miR-126, miR223 und miR-320, und ein hohes Level von miR-28-3p bestimmt wird, Typ-2-Diabetes aufzuweisen.

11. Verfahren gemäß Anspruch 7, wobei ein Individuum durch ein niedriges Level von miR-15a, miR-126, miR223 und miR-320, und ein hohes Level von miR-28-3p, und ein niedriges Level von einer oder mehreren von miR-20b, miR-21, miR-24, miR-29b, miR-191, miR-197, miR-486 und miR-150 bestimmt wird, Typ-2-Diabetes aufzuweisen.

12. Verfahren gemäß Anspruch 6, wobei eine positive Vorhersage von Typ-2-Diabetes durch ein niedriges Level von miR-15a, miR-29b, miR-126 und miR223, und ein hohes Level von miR-28-3p gegeben wird.

## Revendications

1. Procédé de prévision d'un infarctus du myocarde, comprenant la détermination, dans un échantillon obtenu d'un individu, du niveau d'au moins 2 micro-ARN choisis dans le groupe comprenant miR-126, miR-223 et miR-197 ou choisis dans le groupe comprenant miR-126, miR-24 et miR-197.

2. Procédé selon la revendication 1, dans lequel les niveaux de :
i. miR-126 et de miR-223 ; ou de
ii. miR-126 et miR-24
sont déterminés.

3. Procédé selon la revendication 1, dans lequel le niveau de miR-126 et de miR-223 est déterminé.

4. Procédé selon la revendication 2 ou la revendication 3, dans lequel le niveau de miR-197 est en outre déterminé.

5. Procédé de détermination du fait qu'un individu a ou non un diabète de type II, comprenant la détermination dans un échantillon obtenu de l'individu du niveau des micro-ARN suivants : miR-15a, miR-126, miR223, miR-320 et miR-28-3p.

6. Procédé de prévision d'un diabète de type II comprenant la détermination dans un échantillon obtenu d'un individu du niveau des micro-ARN suivants : miR-15a, miR-29b, miR-126, miR223 et miR-28-3p.

7. Procédé selon la revendication 5, comprenant en outre la détermination du niveau d'un ou plusieurs des micro-ARN suivants : miR-20b, miR-21, miR-24, miR-29b, miR-191, miR-197, miR-486 etmiR-150.

8. Procédé selon l'une quelconque des revendications précédentes, dans lequel l'échantillon est un échantillon de tissu, de sang ou de plasma.

9. Procédé selon la revendication 8, dans lequel l'échantillon est le plasma.

10. Procédé selon la revendication 5, dans lequel il est déterminé qu'un individu a un diabète de type II par un faible niveau de miR-15a, miR-126, miR-223 et miR-320 et d'un niveau élevé de miR-28-3p.

11. Procédé selon la revendication 7, dans lequel il est déterminé qu'un individu a un diabète de type II par un faible niveau de miR-15a, miR-126, miR-223 et miR-320 et un niveau élevé de miR-28-3p, et un faible niveau d'un ou plusieurs de miR-20b, miR-21, miR-24, miR-29b, miR-191, miR-197, miR-486 et miR-150.

12. Procédé selon la revendication 6, dans lequel une prévision positive de diabète de type II est donnée par un faible niveau de miR-15a, miR-29b, miR-126 et miR223 et un niveau élevé de miR-28-3p,
